(19) Europäisches Patentamt
European Patent Office
Office européen des brevets

(11) **EP 2 370 069 B1**

(12) **EUROPEAN PATENT SPECIFICATION**

(45) Date of publication and mention
of the grant of the patent:
**30.09.2020   Bulletin 2020/40**

(21) Application number: **09764392.8**

(22) Date of filing: **20.11.2009**

(51) Int Cl.:
*A61K 31/05* (2006.01)     *A61P 1/02* (2006.01)
*A61K 9/70* (2006.01)      *A61K 9/08* (2006.01)
*A61K 9/06* (2006.01)      *A61K 8/34* (2006.01)
*A61K 8/36* (2006.01)      *A61Q 11/00* (2006.01)
*A61K 31/045* (2006.01)    *A61K 31/047* (2006.01)
*A61K 31/192* (2006.01)    *A61K 31/12* (2006.01)

(86) International application number:
**PCT/US2009/065318**

(87) International publication number:
**WO 2010/062835 (03.06.2010 Gazette 2010/22)**

(54) **ANTIOXIDANT COMPOSITIONS FOR SOFT ORAL TISSUE AND METHODS OF FORMULATION AND USE THEREOF**

ANTIOXIDANS-ZUSAMMENSETZUNGEN FÜR WEICHES MUNDGEWEBE UND VERFAHREN ZU IHRER HERSTELLUNG UND VERWENDUNG

COMPOSITIONS ANTI-OXYDANTES POUR TISSU ORAL MOU ET PROCÉDÉS DE FORMULATION ET D'UTILISATION DE CES COMPOSITIONS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **26.11.2008   US 118253 P**
**30.09.2009   US 247356 P**

(43) Date of publication of application:
**05.10.2011   Bulletin 2011/40**

(73) Proprietor: **Perio Sciences, LLC**
**Dallas, Texas 75230 (US)**

(72) Inventors:
• **ZIELINSKI, Jan**
**Vista, California 92081 (US)**
• **MOON, Russell P.**
**Dallas, Texas 75220 (US)**
• **ALLEN, Edward P.**
**Dallas, Texas 75231 (US)**

(74) Representative: **Maiwald Patent- und Rechtsanwaltsgesellschaft mbH**
**Elisenhof**
**Elisenstraße 3**
**80335 München (DE)**

(56) References cited:
WO-A1-2005/004630      WO-A1-2007/042835
WO-A1-2007/107596      WO-A1-2008/120220
WO-A2-00/38620         WO-A2-02/081651
WO-A2-2008/001325      CN-A- 1 504 215
JP-A- 5 117 145        JP-A- 2004 231 602
US-A1- 2007 104 808    US-A1- 2007 225 360
US-A1- 2008 227 867    US-B1- 6 572 882

• KARAKAYA SIBEL: "BIOAVAILABILITY OF PHENOLIC COMPOUNDS", TRANSACTIONS OF THE IRON & STEEL SOCIETY, WARRENDALE, US, vol. 44, no. 6, 1 January 2004 (2004-01-01), pages 453-464, XP008070961, ISSN: 1051-0508

**Description**

TECHNICAL FIELD

The current invention relates to antioxidant compositions for use in the treatment or prevention of an oral disease by topical application to soft oral tissue as defined in the claims.

BACKGROUND

Oxidative damage of soft oral tissues has many causes. It may result from exposure to chemicals or biochemicals, for example by smoking or other tobacco use, teeth whitening products, alcohol, or even from the body's own processes. Chemicals known as free radicals are often the cause of oxidative damage to the body. Free radicals and other reactive species are also known to cause oxidative damage to organisms. As this oxidative damage occurs, it may result in inflammation, periodontal disease, and related problems, such as cardiovascular disease or increased susceptibility to formation of cancerous lesions in the oral cavity. Finally, there are specific pathologies, for example leukoplakia, that are associated with a person's use of tobacco products.

Periodontal disease often begins with oxidative damage to a few oral cells. The body recognizes and tries to clear away these damaged cells in part through the actions of inflammation pathways. Inflammatory pathways, however, are typically not able to only clean up the damaged cells. Other, healthy cells are damaged in the process, often causing much more harm than the original oxidative damage.

Current treatments for periodontal disease involve surgery in the local area or antibiotics if the periodontal disease has progressed far enough to allow infection to set in. These treatments do not address the underlying systemic nature of periodontal disease and instead merely address very specific and local problems. Further, they have limited or no ability to cause regrowth and healing of the diseased tissue. Other problems resulting from oxidative damage of soft oral tissues similarly do not have effective treatments. Accordingly, a need exists for effective methods to treat periodontal disease and other soft oral tissue diseases resulting from oxidative damage or to prevent such periodontal disease all together.

Antioxidants are known to protect organisms, such as humans, from oxidative damage, but their effective administration is a problem. For example, antioxidants ingested in food or as a pill have a limited effect on periodontal disease because the uptake and management of antioxidants is tightly regulated by body mechanisms such that antioxidants are distributed to all body parts, resulting in only a minimal portion of the ingested amount reaching the gingiva and thus limiting the antioxidant effect in that tissue. Similarly, previous antioxidant compositions have been developed for use in specific areas, such as the skin, but these compositions are not suitable for use on soft oral tissue due to the many differences between skin and soft oral tissue. For example, commonly used skin antioxidant compositions are formulated at a pH far too low for use in the oral environment, and will demineralize tooth enamel. There are other issues of compatibility with formulations intended for use on the skin, which frequently contain oils, lipids and detergents that are inappropriate or ineffective in the moist environment of the oral cavity.

Accordingly, a need exists for antioxidant compositions able to topically treat or prevent periodontal disease or other soft oral tissue diseases, particularly diseases resulting from environmentally caused or naturally occurring oxidative damage.

JPH05117145 relates to a collagenase inhibitor for treating periodontal disease comprising caffeic acid or its salt and optionally ascorbic acid or its salt.

CN1504215 A relates to a traditional Chinese medicine formulation for treating oral disease comprising Feilong Zhang blood velamen, Feilong Zhang blood leaves and ferulic acid.

JP 2004/231602 A relates to a buccal composition containing galenical extract having antioxidation activity for prevention/treatment of periodontitis.

SUMMARY

The current invention is directed to oral antioxidant compositions for use in the treatment or prevention of an oral disease as defined in the claims.

The present invention relates to an oral antioxidant composition comprising :

between 0.0001% and 5.0% w/w trans-ferulic acid;
between 0.0001 % and 5.0 % w/w phloretin; and
an orally pharmaceutically acceptable carrier,
wherein the pH of the oral antioxidant composition is at least 5.5
for use in the treatment or prevention of an oral disease by topical application to soft oral tissue.

In more specific embodiments, the oral antioxidant composition may comprise between 0.0001% and 5.0 % w/w of at least one or at least two additional antioxidants. The additional antioxidants may be tetrahydrocurcuminoids, or a stilbene derivative. In specific embodiments, the stilbene derivative may be resveratrol, and the tetrahydrocurcuminoids may comprise between 75% to 90% w/w tetrahydrocurcumin, 15% to 20% w/w tetrahydrodemethoxycurcumin, and between 1% to 4% w/w tetrahydrobisdemethoxycurcumin.

In specific embodiments, the oral antioxidant composition has a pH of between 5.5 and 8.0. The oral antioxidant composition may have a total amount of antioxidant of less than 5% w/w. The oral antioxidant composition may be formulated as a paste, gel, rinse, spray, aerosol spray, syrup, powder, reconstitutable powder, tablet, gum, lipstick, lip balm, lozenge, dental tray, teeth whitening delivery vehicle, pharmaceutical delivery vehicle, or dissolvable strip. The oral antioxidant composition may comprise at least one additional component selected from the group consisting of: solvents, preservatives, moisturizers, bases, viscosity enhancers, surfactants, therapeutic additives, flavor enhancers, color enhancers, water, and any combinations thereof.

The oral disease, in certain embodiments, may be a periodontal disease such as gingivitis or periodontitis. The soft oral tissue, in some embodiments, may be the oral mucosa, gingiva, tongue tissue, periodontal ligament, soft or hard palate, or lip tissue. The antioxidant composition may be reapplied, for example it may be reapplied at least every hour, every 4 hours, every 8 hours, every day, every week, or after additional exposure of the soft oral tissue to an oxidizing chemical or the sun.

The following abbreviations may be used in the specification and drawings: HGF - human gingival fibroblasts

HPDL - human periodontal ligament cells
FBS -fetal bovine serum
DMSO - dimethyl sulfoxide
DEVD - The polypeptide consisting of: Asparagine-Glutamine-Valine-Asparagine
Z-VAD-FMK (carbobenzoxy-valyl-alanyl-aspartyl-[O-methyl]-fluoromethylketone).

Other abbreviations not listed here may also be used.

BRIEF DESCRIPTION OF THE DRAWINGS

Embodiments of the present invention may be better understood through reference to the following figures in which:

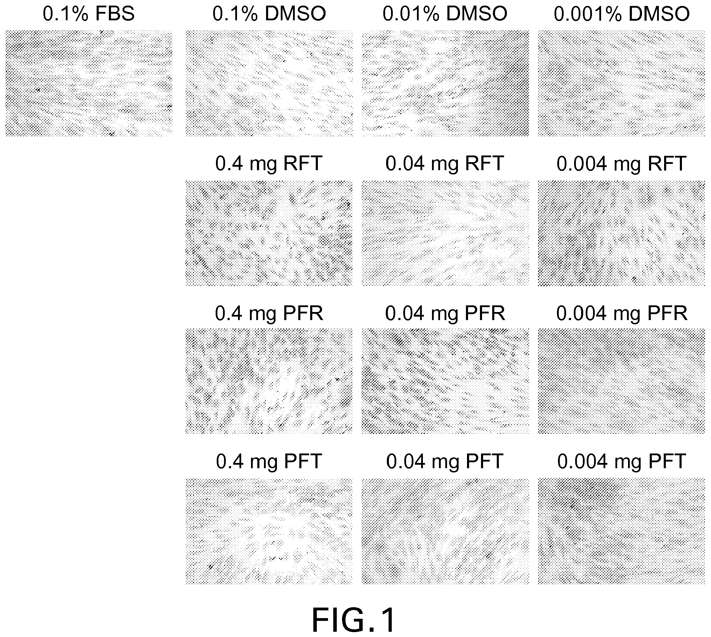

FIGURE 1 shows Giemsa-stained slides of human gingival fibroblast (HGF) cells exposed for 48 hours to 0.4 mg/mL, 0.04 mg/mL, or 0.004 mg/mL (corresponding to the same % w/v) of a 1:1:1 molar combination of resveratrol, trans-ferulic acid, and tetrahydrcurcuminoids CG™ (RFT), phloretin, trans-ferulic acid, and resveratrol (PFR), or phloretin, trans-ferulic acid, and tetrahydrocurcuminoids CG™ (PFT) in 0.1% FBS serum. Control samples were treated with 0.1% fetal bovine serum (FBS) or DMSO in various concentrations alone.

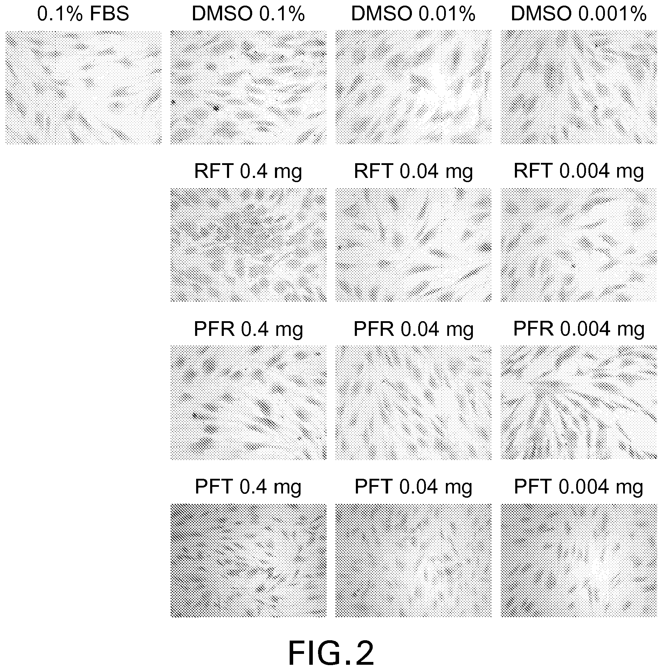

FIGURE 2 shows Giemsa-stained slides of human periodontal ligament (HPDL) cells exposed for 48 hours to 0.4 mg/mL, 0.04 mg/mL, or 0.004 mg/mL (corresponding to the same % w/v) of a 1:1:1 molar combination of resveratrol, trans-ferulic acid, and tetrahydrcurcuminoids CG™ (RFT), phloretin, trans-ferulic acid, and resveratrol (PFR), or phloretin, trans-ferulic acid, and tetrahydrocurcuminoids CG™ (PFT) in 0.1% FBS serum. Control samples were treated with 0.1% fetal bovine serum (FBS) or DMSO in various concentrations alone.

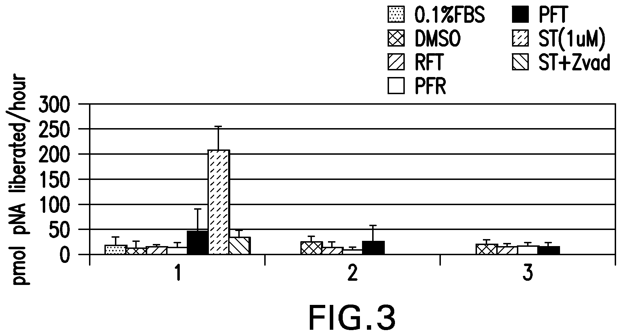

FIGURE 3 shows the effects on apoptosis of exposure of HGF as determined by caspase activity after 48 hours of exposure to $10^{-3}$ M, $10^{-4}$ M, or $10^{-5}$ M of a 1:1:1 molar combination of resveratrol, trans-ferulic acid, and tetrahydrcurcuminoids CG™ (RFT), phloretin, trans-ferulic acid, and resveratrol (PFR), or phloretin, trans-ferulic acid, and tetrahydrocurcuminoids CG™ (PFT) in 0.1% FBS serum. Cells were also exposed to 1 μM Staurosporine (st), an apoptosis inducer, alone or with the apoptosis inhibitor, DEVD-Z-VAD-FMK (st+zvad) (50 μM).

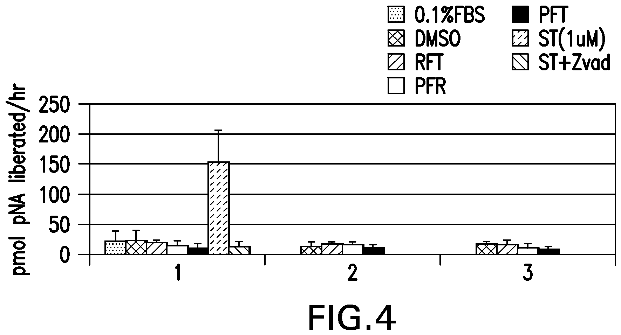

FIGURE 4 shows the effects on apoptosis of exposure of HPDL as determined by caspase activity after 48 hours of exposure to $10^{-3}$ M, $10^{-4}$ M, or $10^{-5}$ M of a 1:1:1 molar combination of resveratrol, trans-ferulic acid, and tetrahydrcurcuminoids CG™ (RFT), phloretin, trans-ferulic acid, and resveratrol (PFR), or phloretin, trans-ferulic acid, and tetrahydrocurcuminoids CG™ (PFT) in 0.1% FBS serum. Cells were also exposed to 1 μM Staurosporine (st), an apoptosis inducer, alone or with the apoptosis inhibitor, DEVD-Z-VAD-FMK (st+zvad) (50 μM).

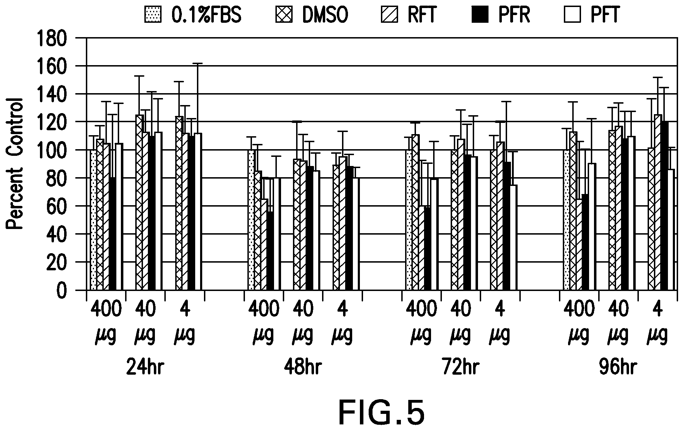

FIGURE 5 presents the effects on cell proliferation of HGF for 24 hours, 48 hours, 72 hours, or 96 hours to 0.4 mg/mL, 0.04 mg/mL, or 0.004 mg/mL (corresponding to the same % w/v) of a 1:1:1 molar combination of resveratrol, trans-ferulic acid, and tetrahydrcurcuminoids CG™ (RFT), phloretin, trans-ferulic acid, and resveratrol (PFR), or phloretin, trans-ferulic acid, and tetrahydrocurcuminoids CG™ (PFT) in 0.1% FBS serum. Control samples were treated with 0.1% fetal bovine serum (FBS) or DMSO in various concentrations alone.

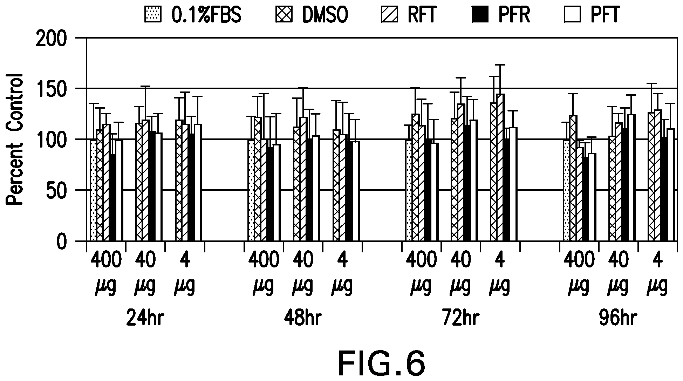

FIGURE 6 presents the effects on cell proliferation of HPDL for 24 hours, 48 hours, 72 hours, or 96 hours to 0.4 mg/mL, 0.04 mg/mL, or 0.004 mg/mL (corresponding to the same % w/v) of a 1:1:1 molar combination of resveratrol, trans-ferulic acid, and tetrahydrcurcuminoids CG™ (RFT), phloretin, trans-ferulic acid, and resveratrol (PFR), or phloretin, trans-ferulic acid, and tetrahydrocurcuminoids CG™ (PFT) in 0.1% FBS serum. Control samples were treated with 0.1% fetal bovine serum (FBS) or DMSO in various concentrations alone.

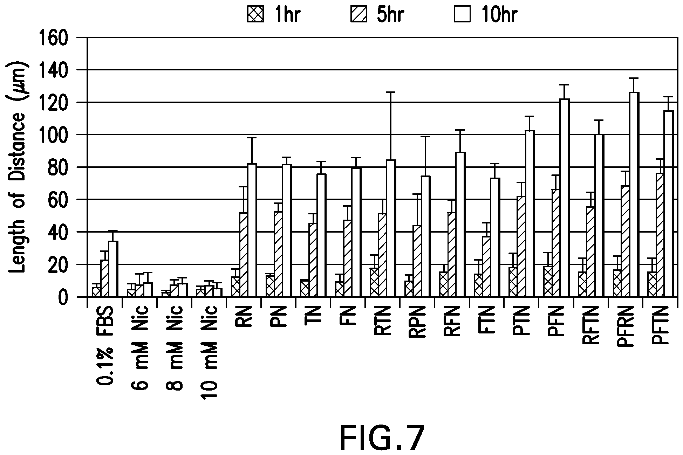

FIGURE 7 shows the effects on cell migration of exposure of HGF to 6 mM nicotine for two hours followed by exposure to $10^{-5}$ M resveratrol (RN), phloretin (PN), tetrahydrocurcuminoids CG™ (TN), or trans-ferulic acid (FN), or a 1:1 or 1:1:1: molar ratio of resveratrol and tehatrhydrocurcuminoids CG™ (RTN), resveratrol and phloretin (RPN), resveratrol and trans-ferulic acid (RFN), trans-ferulic acid and thetrahydrocurcuminoids CG™ (FTN), phloretin and tetrahydrocurcuminoids CG™ (PTN), phloretin and trans-ferulic acid (PFN), resveratrol, trans-ferulic acid, and tetrahydrocurcuminoids CG™ (RFTN), phloretin, trans-ferulic acid, and resveratrol (PFRN), or phloretin, trans-ferulic acid, and tetrahydrocurcuminoids CG™ (PFTN). Control samples were treated with 0.1% fetal bovine serum (FBS) or nicotine in various concentrations alone.

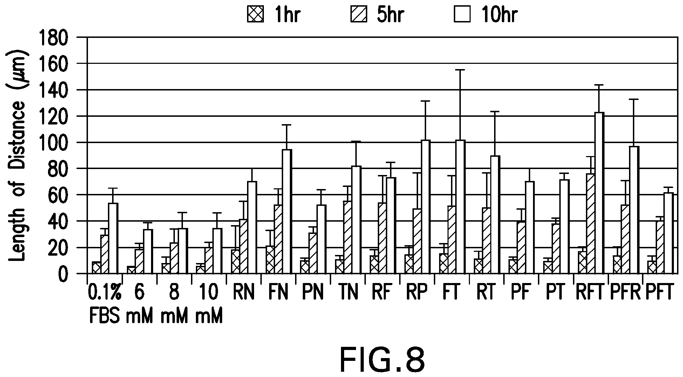

FIGURE 8 shows the effects on cell migration of exposure of HDPL to 6 mM nicotine for two hours followed by exposure to $10^{-5}$ M resveratrol (RN), phloretin (PN), tetrahydrocurcuminoids CG™ (TN), or trans-ferulic acid (FN), or a 1:1 or 1:1:1 molar ratio of resveratrol and tehatrhydrocurcuminoids CG™ (RTN), resveratrol and phloretin (RPN), resveratrol and trans-ferulic acid (RFN), trans-ferulic acid and tetrahydrocurcuminoids CG™ (FTN), phloretin and tetrahydrocurcuminoids CG™ (PTN), phloretin and trans-ferulic acid (PFN), resveratrol, trans-ferulic acid, and tetrahydrocurcuminoids CG™ (RFTN), phloretin, trans-ferulic acid, and resveratrol (PFRN), or phloretin, trans-ferulic acid, and tetrahydrocurcuminoids CG™ (PFTN). Control samples were treated with 0.1% fetal bovine serum (FBS) or nicotine in various concentrations alone.

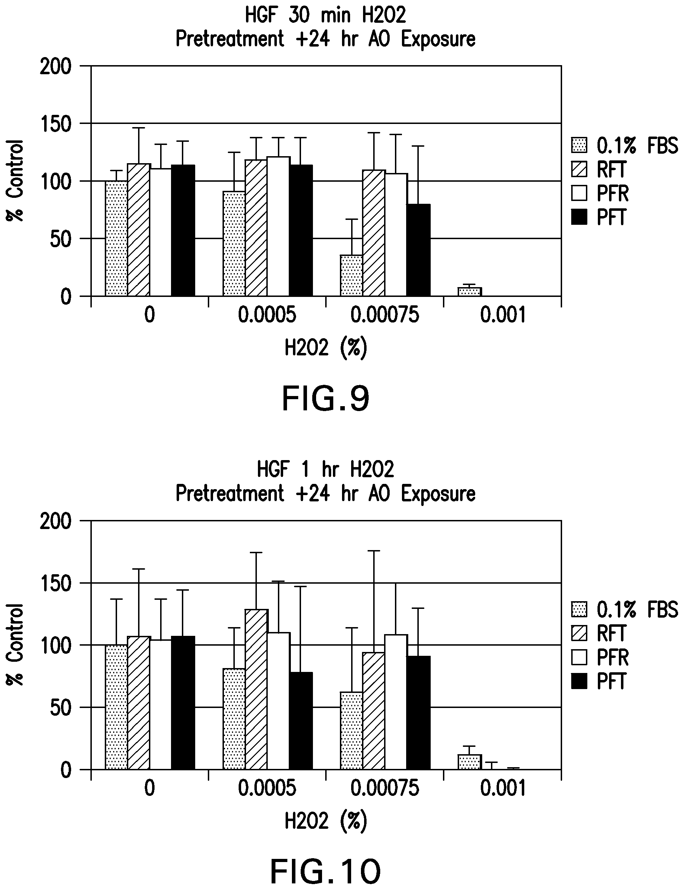

FIGURE 9 shows the effects on cell death of exposure of HGF to indicated percentages of hydrogen peroxide ($H_2O_2$) for 30 minutes followed by exposure to $10^{-5}$ M antioxidant compositions for 24 hours. Antioxidant combinations tested include resveratrol, tetrahydrocurcuminoids CG™, and trans-ferulic acid (RFT), phloretin, trans-ferulic acid, and resveratrol (PFR), and phloretin, trans-ferulic acid, and tetrahydrocurcuminoids CG™ (PFT). Control samples were treated with 0.1% fetal bovine serum (FBS) alone.

FIGURE 10 shows the effects on cell death of exposure of HGF to indicated percentages of hydrogen peroxide ($H_2O_2$) for one hour (60 minutes) followed by exposure to $10^{-5}$ M antioxidant compositions for 24 hours. Antioxidant combinations tested include resveratrol, tetrahydrocurcuminoids CG™, and trans-ferulic acid (RFT), phloretin, trans-ferulic acid, and resveratrol (PFR), and phloretin, trans-ferulic acid, and tetrahydrocurcuminoids CG™ (PFT). Control samples were treated with 0.1% fetal bovine serum (FBS) alone.

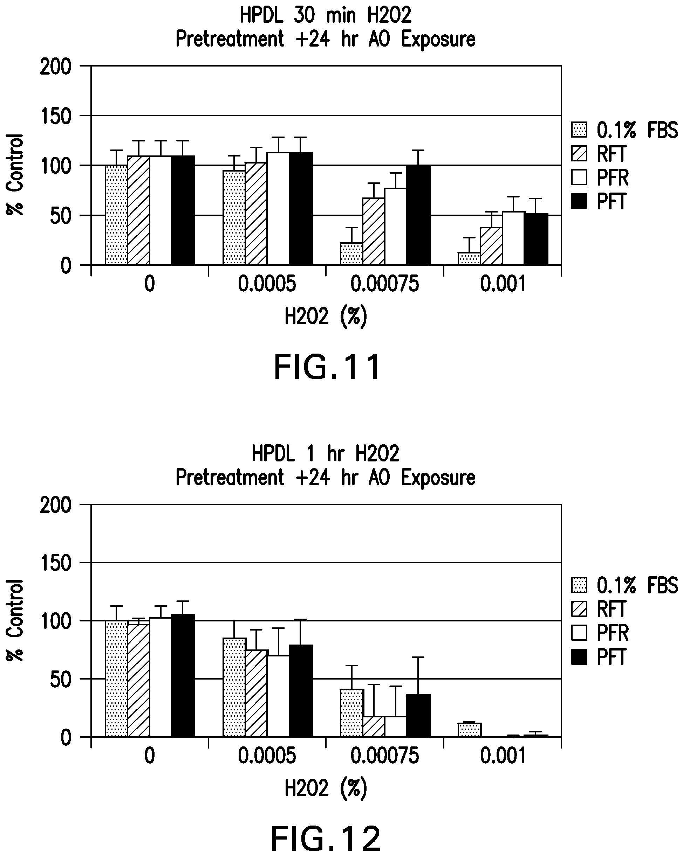

FIGURE 11 shows the effects on cell death of exposure of HPDL to indicated percentages of hydrogen peroxide ($H_2O_2$) for 30 minutes followed by exposure to $10^{-5}$ M antioxidant compositions for 24 hours. Antioxidant combinations tested include resveratrol, tetrahydrocurcuminoids CG™, and trans-ferulic acid (RFT), phloretin, trans-ferulic acid, and resveratrol (PFR), and phloretin, trans-ferulic acid, and tetrahydrocurcuminoids CG™ (PFT). Control samples were treated with 0.1% fetal bovine serum (FBS) alone.

FIGURE 12 shows the effects on cell death of exposure of HPDL to indicated percentages of hydrogen peroxide ($H_2O_2$) for one hour (60 minutes) followed by exposure to $10^{-5}$ M antioxidant compositions for 24 hours. Antioxidant combinations tested include resveratrol, tetrahydrocurcuminoids CG™, and trans-ferulic acid (RFT), phloretin, trans-ferulic acid, and resveratrol (PFR), and phloretin, trans-ferulic acid, and tetrahydrocurcuminoids CG™ (PFT). Control samples were treated with 0.1% fetal bovine serum (FBS) alone.

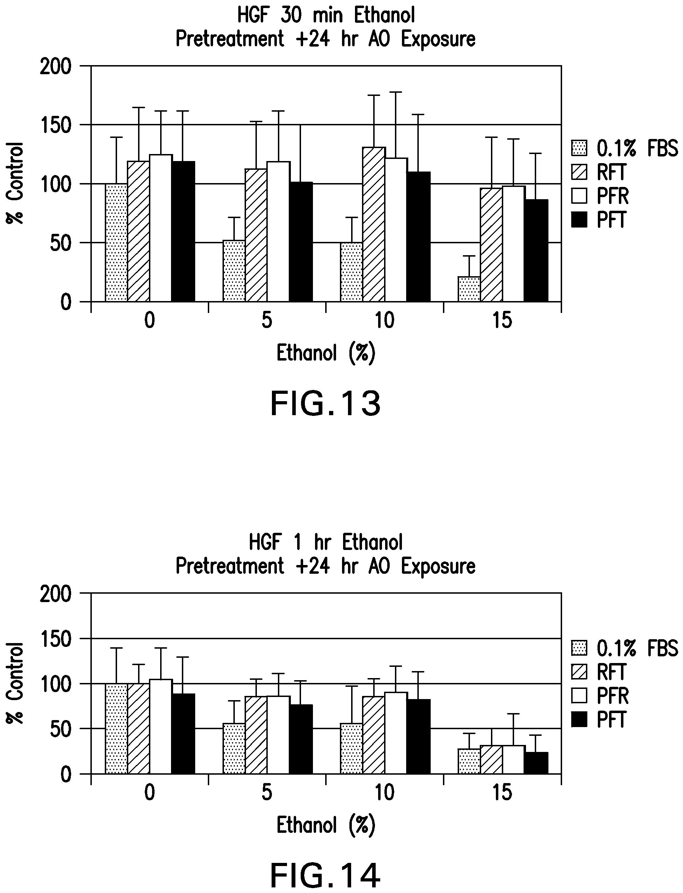

FIGURE 13 shows the effects on cell death of exposure of HGF to indicated percentages of ethanol for 30 minutes followed by exposure to $10^{-5}$ M antioxidant compositions for 24 hours. Antioxidant combinations tested include resveratrol, tetrahydrocurcuminoids CG™, and trans-ferulic acid (RFT), phloretin, trans-ferulic acid, and resveratrol (PFR), and phloretin, trans-ferulic acid, and tetrahydrocurcuminoids CG™ (PFT). Control samples were treated with 0.1% fetal bovine serum (FBS) alone.

FIGURE 14 shows the effects on cell death of exposure of HGF to indicated percentages of ethanol for one hour (60 minutes) followed by exposure to $10^{-5}$ M antioxidant compositions for 24 hours. Antioxidant combinations tested include resveratrol, tetrahydrocurcuminoids CG™, and trans-ferulic acid (RFT), phloretin, trans-ferulic acid, and resveratrol (PFR), and phloretin, trans-ferulic acid, and tetrahydrocurcuminoids CG™ (PFT). Control samples were treated with 0.1% fetal bovine serum (FBS) alone.

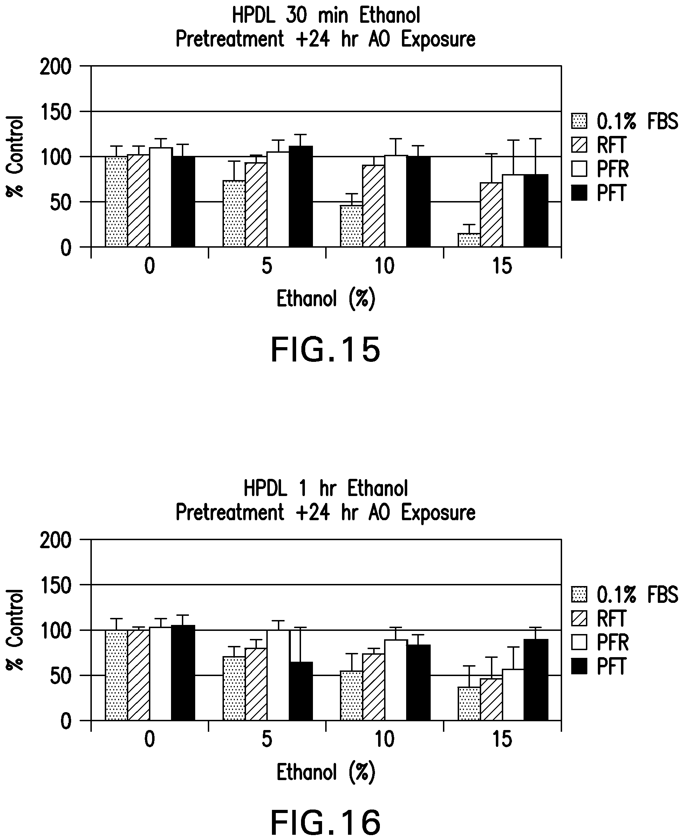

FIGURE 15 shows the effects on cell death of exposure of HPDL to indicated percentages of ethanol for 30 minutes followed by exposure to $10^{-5}$ M antioxidant compositions for 24 hours. Antioxidant combinations tested include resveratrol, tetrahydrocurcuminoids CG™, and trans-ferulic acid (RFT), phloretin, trans-ferulic acid, and resveratrol (PFR), and phloretin, trans-ferulic acid, and tetrahydrocurcuminoids CG™ (PFT). Control samples were treated with 0.1% fetal bovine serum (FBS) alone.

FIGURE 16 shows the effects on cell death of exposure of HPDL to indicated percentages of ethanol for one hour (60 minutes) followed by exposure to $10^{-5}$ M antioxidant compositions for 24 hours. Antioxidant combinations tested include resveratrol, tetrahydrocurcuminoids CG™, and trans-ferulic acid (RFT), phloretin, trans-ferulic acid, and resveratrol (PFR), and phloretin, trans-ferulic acid, and tetrahydrocurcuminoids CG™ (PFT). Control samples were treated with 0.1% fetal bovine serum (FBS) alone.

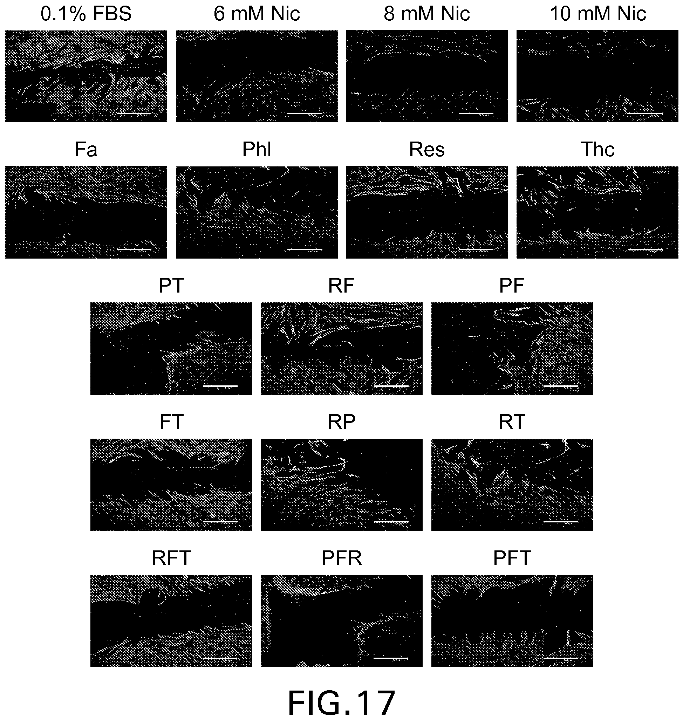

FIGURE 17 shows representative photomicrographs illustrating the effects of various antioxidants or antioxidant combinations at $10^{-5}$ M concentration on Rac-GTP expression in HGF cells exposed to nicotine. Treatments are abbreviated as follows: 0.1% FBS (no nicotine, no antioxidants); Nic = nicotine hemi sulfate; Fa=trans-ferulic acid; Phl=phloretin; Res=resveratrol; Thc=tetrahydrocurcuminoids CG™; in double and triple combinations (P= phloretin; T= tetrahydrocurcuminoids CG™; F = trans-ferulic acid; and R= resveratrol).

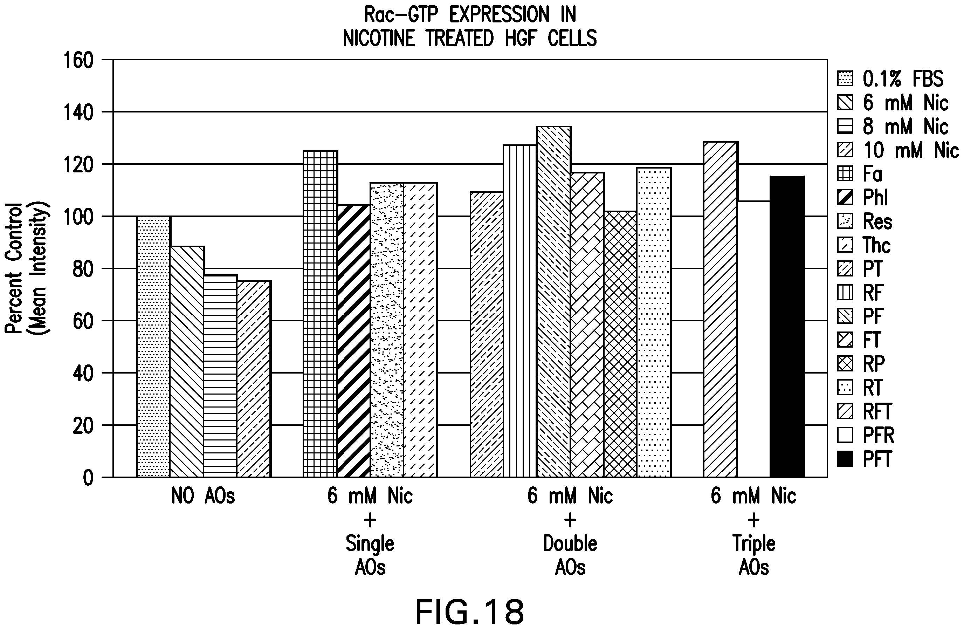

FIGURE 18 presents the overall effects of antioxidants on Rac-GTP expression in HGF cells exposed to nicotine. Treatments are abbreviated as follows: 0.1% FBS (no nicotine, no antioxidants); Nic = nicotine hemi sulfate; Fa=trans-ferulic acid; Phl=phloretin; Res=resveratrol; Thc=tetrahydrocurcuminoids CG™; in double and triple combinations (P= phloretin; T= tetrahydrocurcuminoids CG™; F = trans-ferulic acid; and R= resveratrol).

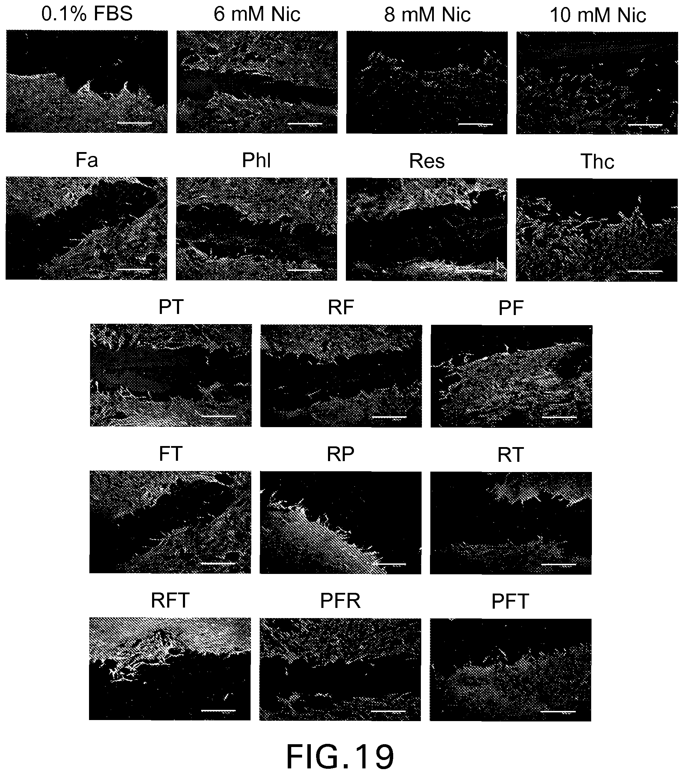

FIGURE 19 shows representative photomicrographs illustrating the effects of various antioxidants or antioxidant combinations at $10^5$ M concentration on Rac-GTP expression in HPDL cells exposed to nicotine. Treatments are abbreviated as follows: 0.1% FBS (no nicotine, no antioxidants); Nic = nicotine hemi sulfate; Fa=trans-ferulic acid; Phl=phloretin; Res=resveratrol; Thc=tetrahydrocurcuminoids CG™; in double and triple combinations (P= phloretin; T= tetrahydrocurcuminoids CG™; F = trans-ferulic acid; and R= resveratrol).

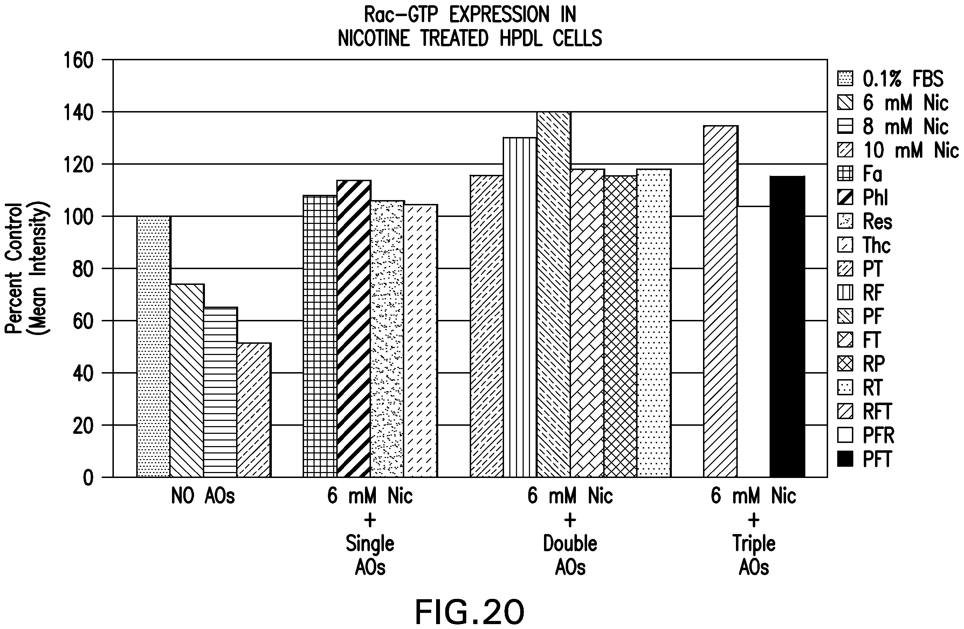

FIGURE 20 presents the overall effects of antioxidants on Rac-GTP expression in HPDL cells exposed to nicotine.Treatments are abbreviated as follows: 0.1% FBS (no nicotine, no antioxidants); Nic = nicotine hemi sulfate; Fa=trans-ferulic acid; Phl=phloretin; Res=resveratrol; Thc=tetrahydrocurcuminoids CG™; in double and triple combinations (P= phloretin; T= tetrahydrocurcuminoids CG™; F = trans-ferulic acid; and R= resveratrol).

## DETAILED DESCRIPTION

The current invention relates to antioxidant compositions for use in the treatment or prevention of an oral disease as defined in the claims.

Discussed herein are various antioxidants that are components of the antioxidant compositions of the invention. Although some derivatives of these antioxidants are identified specifically herein, this is not intended to in any way exclude other derivatives of these antioxidants from the scope of the invention. According to embodiments of the invention, any derivatives of the antioxidants discussed herein that also have antioxidant properties may be substituted for the named antioxidant unless it is clear from context that such substitution is not intended. Further, antioxidants as discussed herein may, unless otherwise clear from context, include combinations of the named antioxidant and derivatives or combinations of derivatives alone. Similarly, when groups of antioxidants are discussed, combinations of group members may be used unless otherwise indicated by context.

*Antioxidants*

Antioxidant compositions of the invention comprise trans-ferulic acid and phloretin but they may also comprise combinations of three or more antioxidants. According to some embodiments, combinations of multiple antioxidants may exhibit synergistic effects. For example, in the data presented in the Examples, combinations of two or three antioxidants generally perform far better in reducing or reversing the effects of oxidative damage than single antioxidants. In one embodiment, all four antioxidants, a cinnamic acid derivative, phloretin, tetrahydrocurcuminoids, and a stilbene derivative, may all be included in the antioxidant composition to provide a more enhanced synergistic effect.

According to the invention, an antioxidant of the antioxidant composition for use in the current invention includes phloretin or dihydrochalcone phloretin. Without limiting the mode of action of the invention, phloretin may exert an antioxidant effect by reducing reactive free radicals, like reactive oxygen and reactive nitrogen species.

According to the invention , an antioxidant of the antioxidant composition for use in the invention is the cinnamic acid derivative trans-ferulic acid (including its antioxidant pharmacore 2,6-dihydroxyacetophenome), caffeic acid, p-coumaric acid, and sinapinic acid. Without limiting the mode of action of the invention, cinnamic acids may neutralize free radicals.

According to another embodiment, an antioxidant for use in the antioxidant composition in the current invention may be tetrahydrocurcuminoids (derived from rhizomes of *Curcuma longa*). An active compound found in *Curcuma longa* is curcumin. Curcumin has been found to act as a potent antioxidant. Tetrahydrocurcumin, a metabolite of curcumin, has been found to be a more potent antioxidant and more stable compared to curcumin. Tetrahydrocurcumin is available commercially, for example, it is the main component of Tetrahydrocurcuminoids CG™ as sold by Sabinsa Corp. (Piscataway, NJ). Tetrahydrocurcuminoids CG™ contains on a w/w basis tetrahydrocurcumin (75-90%), tetrahydrodemethoxycurcumin (15-20%), and tetrahydrobisdemethoxycurcumin (1-4%). Each of these components is a potent antioxidant. Accordingly, in some embodiments, curcumin or tetrahydrocurcumin may be used in place of tetrahydrocurcuminoids. Further, each component of Tetrahydrocurcuminoids CG™ may be used separately as tetrahydrocurcuminoids. Tetrahydrocurcuminoids CG™ or other useful tetrahydrocurcuminoids are described in WO 00/61162. Without limiting the mode of action of the invention, tetrahydrocurcuminoids may reduce free radicals.

According to still another embodiment, an antioxidant for use in the antioxidant composition in the current invention may be a stilbene derivative, such as resveratrol. Resveratrol may include, but is not limited to 3,5,4'-trihydroxystilbene, 3,4,3',5'-tetrahydroxystilbene (piceatannol), 2,3',4,5'-tetrahydroxystilbene (oxyresveratrol), 4,4'-dihydroxystilbene, and alpha and beta glucoside, galactoside and mannoside derivatives thereof. Other derivatives are recited in U.S. 6,572,882,. Additionally, analogs of resveratrol such as the 3,4,4',5-tetrahydroxystilbene of U.S. 6,790,869 may also be used. Both cis and trans configurations of resveratrol or its derivatives may be used. Without limiting the mode of action of the invention, resveratrol may neutralize free radicals.

Other antioxidants may also be included in the compositions in some embodiments of the invention. Other antioxidants may include, for example, ascorbic acid, retinol, tocopherol, polyphenolic flavanoids, such as epigallocatechin gallate, and sulfur-based antioxidants such as methionine or lipoic acid.

*Antioxidant Compositions*

Antioxidants as described above may be formulated for topical oral use. Oral use includes application topically to any soft oral tissue, including, but not limited to, the lips, oral mucosa, gingiva, tongue, other oral epithelium, including but not limited to the oral epithelium, oral sulcular epithelium and junctional epithelium, periodontal ligament, soft palate, and hard, and the like. Topical application may, in some embodiments, provide the antioxidants to both the oral epithelium and underlying tissues such as the periodontal ligament, lamina propria and submucosal layers, including mesenchymal and periodontal ligament cells, and gingival fibroblasts. Formulations suitable for oral use include, but are not limited to pastes, gels, rinses, sprays, including aerosol sprays, syrups, powders, including reconstitutable powders, tablets, gums, lipsticks or balms, lozenges, dental trays or other teeth whitening delivery methods, pharmaceutical delivery vehicles such as liposomes, nano-particles, polymer-based delivery systems, and other cellular delivery vectors, particularly vehicles able to penetrate the oral epithelium, and in dissolvable strips. Example dissolvable strips and edible films in which the antioxidant composition may be incorporated are described in U.S. 6,923,981, incorporated by reference herein.

Antioxidant compositions may be applied regularly, for example at least every 4 hours, every 12 hours, 24 hours, 48 hours, 72 hours, or 96 hours, or following each exposure to oxidative agents. Time between applications may vary depending on the antioxidant or antioxidant combinations used, but may be approximately the duration for which the composition causes a desirable effect, such as a cell proliferation effect, increased migration of a cell, or decreased cell death, in a soft oral tissue to which the composition is applied. Antioxidant compositions may be administered repeatedly, for example until the underlying periodontal disease shows sufficient improvement, or on a regularly occurring basis for tobacco users, such as after tobacco use. Antioxidant compositions may be administered on a prophylactic basis in the absence of any evidence of clinical symptoms. The duration of administration may vary depending on the formulation of the antioxidant compositions. For example, the composition may be allowed to dissipate naturally, or it may be applied for a set period of time. For example a more viscous formulation may be applied for a set time in a professional setting, such as after teeth cleaning.

The antioxidant compositions may preferably have a pH higher than skin antioxidant preparations. This provides the added benefit of avoiding lower pHs, which may damage enamel on the teeth. Example antioxidant compositions for oral use have a pH of 5.5, the normal lower limit of salivary pH, or higher. In some examples, the pH may be as high as 6.0 to further avoid demineralization of the teeth. Antioxidant compositions according to some embodiments may have a pH of up to around 7.4, the upper normal limit of salivary pH, or lower. Saliva is a buffer, so small quantities of materials, such as antioxidant compositions, introduced into the mouth may adjust to salivary pH or near salivary pH. Accordingly, some antioxidant compositions may have a pH near, but outside of salivary pH range. In some compositions, the upper pH limit may be set by the pH at which one of the antioxidants in the composition becomes predominantly its salt, typically around pH 8. According to one specific embodiment, antioxidant compositions may have a pH of between 5.5 and 7.4, similar to salivary pH. In another embodiment, the antioxidant composition may have a pH of between 5.5 and 8.0, between 5.5 and 7.0, between 5.5 and 6.5, between 5.5 and 6.0, between 6.0 and 8.0, between 6.0 and 7.0, or between 6.0 and 6.5.

The antioxidant compositions of the invention comprise trans-ferulic acid and phloretin and may comprise tetrahydrocurcuminoids, such at tetrahydrocurcuminoids CG™, or a stilbene derivative, such as resveratrol, as well as other antioxidants. However, according to some embodiments, antioxidant compositions may consist essentially of trans-ferulic acid and phloretin and a pharmaceutically acceptable carrier. Other antioxidants may be absent from such compositions or present only in trace amounts, such as a by-product, unable to cause any substantial therapeutic or preventative effect.

In particular, the following antioxidant combinations either comprising additional antioxidants or consisting essentially of only the indicated antioxidants, with pharmaceutically acceptable carriers, are contemplated as embodiments of the current invention:

Phloretin + Cinnamic Acid Derivative (such as Trans-Ferulic Acid)

Phloretin + Cinnamic Acid Derivative (such as Trans-Ferulic Acid) + Tetrahydrocurcuminoids (such as Tetrahydrocurcuminoids CG™)

Phloretin + Cinnamic Acid Derivate (such as Trans-Ferulic Acid) + Stilbene Derivative (such as Resveratrol)

Phloretin + Cinnamic Acid Derivative (such as Trans-Ferulic Acid) + Tetrahydrocurcuminoids (such as Tetrahydrocurcuminoids CG™) + Stilbene Derivative (such as Resveratrol).

According to particular embodiments, the concentration of each individual antioxidant in the above compositions may be above 0.0001% w/w, above 0.001% w/w, above 0.01% w/w, as high as 0.5% w/w, 1.0%, w/w, 1.5% w/w, 2% w/w, 3% w/w, 4% w/w, 5%, w/w and in ranges between any combinations of these concentration limits. Expressed another way, the molarity of each antioxidant may be on the order of $10^{-3}$ M or less, $10^{-4}$ M or less, $10^{-5}$ M or less, $10^{-6}$ M or less. Antioxidant concentrations in antioxidant compositions of the invention may be higher than concentrations tested in cell culture, such as in the Examples, due to dilution of the antioxidant in actual use where it is likely combined with saliva and due to shorter exposure times of the actual soft oral tissue as compared to cell culture conditions due to swallowing or other removal of the antioxidant composition.

According to a particular embodiment, the total antioxidant concentration of all antioxidants in an antioxidant composition may be above 0.0001% w/w, above 0.001% w/w, above 0.01% w/w, as high as 0.5% w/w, 1.0%, w/w, 1.5% w/w, 2% w/w, 3% w/w, 5%, w/w, 10% w/w, 15% w/w, or 20% w/w and in ranges between any combinations of these concentration limits.

The concentration of various antioxidant components may be determined by the amount that achieves a desired effect, such as a cell proliferation effect, increased migration of a cell, or decreased cell death, in a soft oral tissue. For some combinations, upper limits on the concentration of the antioxidant components may apply due to decreases in desired effects at high antioxidant concentrations. In other embodiments, upper limits of antioxidant concentration may be set by acceptable levels to avoid gingival hyperplasia.

For antioxidant compositions containing mixtures of antioxidants, the ratios of one antioxidant to another may be mole per mole 1:1. In other embodiments, the ratios may be adjusted depending on the particular antioxidant compositions used. For example, one antioxidant composition may be in the majority and the other may be in the minority due to various factors including differential therapeutic or preventative effects, differential adverse effects, ease of formulation, or cost. Each antioxidant may be present in sufficient amount to cause some improvement in the therapeutic or preventative effects of the antioxidant composition as compared to an identical composition lacking that antioxidant. In alternative embodiments, antioxidants may be present in at least a minimal amount to cause another desirable effect, such as stabilization of another antioxidant.

The antioxidant compositions of the invention may optionally further contain additional materials in some embodiments, such as solvents, surfactants, preservatives, viscosity enhancers, therapeutic additives, flavor or color enhancers, and water. These additional components, excluding water, may be in total up to 5%, 10%, 20%, 30%, 40%, or 50 % w/w of the composition. Individually, additional components, excluding water, may be between 0.5% to 2 % w/w of the composition or as much as 10% w/w or 20 % w/w of the composition. Antioxidant compositions may be up to 95% water, up to 90% water, or between 50% and 90% water.

The antioxidant compositions of the invention may include various solvents. For example, they may include aqueous solvents, organic solvents, including nonaqueous organic solvents and aqueous organic solvents. In particular embodiments, propylene glycol, polyethylene glycol (PEG), such as PEG 600, glycerine, and ethyl alcohol may be used as solvents.

Buffers may be used to maintain pH of the composition. In a particular embodiment, a buffer may be used to maintain a pH of at least 5.0 or between 5.5 and 7.4. Buffers suitable for these pH ranges may include sodium citrate and citric acid buffer, which may be present in an amount of between 0.001 % w/w to about 0.2% w/w.

Preservatives may be added to the antioxidant composition of the invention and include antibacterial compositions or any other preservative used in oral products. Exemplary preservatives include sodium bisulfite, sodium metabisulfite, phenoxyethanol, parabens such as methyl, ethyl, propyl, butyl or isobutyl parabens, 4-hydroxy benzoic acid, benzoic acid, sorbic acid, methyl salicylate, menthol, thymol, eucalyptol, xylitol, and the like.

Viscosity enhancers may be added to the antioxidant compositions of the invention and include any viscosity enhancers used in oral products. Specifically, gelling polymer is a viscosity enhancer and may include cellulose gum, such as carboxymethylcellulose (e.g. pre-hydrated Ticalose® CMC 2500) xanthan gum, gum arabic, guar gum, polyvinyl alcohol, sodium alginate, polyvinylpyrrolidone, sodium hyaluronate, pullulen, carrageenans, and the like. Dextrans, dextran derivatives, or hyaluronic acid may be added to gelling polymers. Thickeners may also be selected to help dissolution of hydrophobic components or stabilize a gel structure. Exemplary thickeners include sugar alcohols such as sorbitol and xylitol, and the like.

Surfactants may be included if a hydrophobic antioxidant or other agent is present in the antioxidant composition. Suitable surfactants include ionic surfactants such as sodium lauryl sulfate, magnesium sodium lauryl sulfate, or tauranol; and nonionic surfactants such as polyoxyethylene sorbitan esters, like Polysorbate 80, polyethylene-polypropylene glycols (particularly Poloxamer® 407), and the like.

Therapeutic additives may be added to the antioxidant compositions of the invention and include other drugs that increase or supplement the antioxidant effect or that are otherwise beneficial to soft oral tissues. For example, a fluoride source beneficial to the teeth may be added. Other wound healing agents, such as polysaccharides and aminopolysaccharides may also be added. Such agents may also help in forming gels, pastes and other formulations. Specific additives may include stannous fluoride, sodium fluoride, triclosan, sodium bicarbonate, chlorhexidine, or a HMG-CoA Reductase Inhibitor, such as a statin or a monocolin.

Flavor or color enhancers may be added to the antioxidant compositions of the invention and include any such materials used in oral cosmetic formulations or topical oral products. For example, flavors commonly used in toothpaste or lipstick or balm may be added. In embodiments where the antioxidant composition is contained in a lipstick or balm, a colorant may be added.

Other additional ingredients include, but are not limited to: potassium nitrate, sodium monofluorophosphate, sodium benzoate, sodium phosphate, aloe vera, lactoferrin, lysozyme, lactoperoxidase, glucose oxidase, mutanase, and dextranase.

Distilled or deionized water may be used to complete the antioxidant composition. Water may be present in much higher levels than other non-antioxidant components.

According to other embodiments, the antioxidant compositions may be in any pharmaceutically acceptable carrier formulated for oral use. Pharmaceutically acceptable carriers commonly include buffers such as citric acid and sodium citrate, and other organic acids, low-molecular weight polypeptides; proteins such as serum albumin, gelatin and immunoglobulin, hydrophilic polymers such as polyvinylpyrrolidone; amino acids such as glycine, glutamine, arginine or lysine; monosaccharides such as mannose, disaccharides, and other carbohydrates; chelating factors such as EDTA; metal ions such as zinc, cobalt or copper; sugar alcohols such as mannitol or sorbitol; and salt-forming counter ions such as sodium. Excipients and diluents may also be present and may include magnesium stearate, calcium carbonate, starch-gelatin paste, talc, aluminum salt, physiological salt solution, lactose, dextrose, sucrose, sorbitol, mannitol, calcium silicate, cellulose, methyl cellulose, amorphous cellulose, polyvinylpyrolidine, methylhydroxy benzoate, and propylhydroxybezoate.

Certain additional components that are formulation-specific may also be added. For example, paste formulations may include one or more ingredients used in oral pastes, such as toothpaste. Examples ingredients include thickening agents such as methylcellulose. carboxymethylcellulose, such as pre-hydrated Ticalose® CMC 2500, or hydroxypropyl methylcellulose and humectants, gel carriers such as gelatin, polyethylene glycol, xanthan gum, gum arabic, and guar gum.

According to some embodiments, the antioxidant compositions of the invention may consist essentially of one or more of the antioxidants and one or more of the above additional materials.

Other embodiments of the invention relate to products containing antioxidants. The products may contain concentrations of the antioxidants identified for antioxidant compositions above. Products may be sold containing these concentrations, or they may be made by adding antioxidants to achieve the above concentrations after sale. For example, one embodiment of the invention relates to antioxidant compositions intended for addition to other products to achieve the antioxidant concentrations described above. These concentrated antioxidant additives may have higher concentrations of antioxidants than described above to allow for their dilution when added to a product.

Example products that may constitute embodiments of the invention or to which concentrated antioxidant additives may be added include existing oral care products, including, but not limited to, pastes, gels, rinses, sprays, including aerosol sprays, syrups powders, including reconstitutable powders, tablets, gums, lipsticks or balms, lozenges, dental trays or other teeth whitening delivery methods, pharmaceutical delivery vehicles such as liposomes, nanoparticles, polymer-based delivery systems, and cellular delivery vectors, and dissolvable strips. In particular, the antioxidant composition may be added to mouthwashes, chewing gum, breath fresheners, lipstick or lip balm, toothpaste, dental floss, fluoride rinses, teeth whitener kits, and the like. Antioxidant compositions may be formulated such that appropriate concentrations of antioxidants or other ingredients are present after addition to another product.

In one particular embodiment, the antioxidant composition may be included in a film-forming gel, such as a primarily water-based, pH-sensitive polymer-containing mucoadhesive that forms a thin, dry film when applied topically. The gel may adhere to a mucosal surface for a time after application, enhancing delivery of antioxidant compositions in some embodiments. The gel may be non-irritating, dry, non-oily, or non-sticky or may be made from Generally Regarded As Safe materials.

Both the carrier non-antioxidant components of the antioxidant composition and volume delivered in each use may be selected based on a variety of factors, including but not limited to the mode of delivery, the form or concentration in which the antioxidants are to be supplied before formulation into the antioxidant composition and the ability or need to administer a precise amount of antioxidants.

Antioxidant compositions may be prepared in any method able to achieve a final composition containing all of the components. One of ordinary skill in the art will appreciate that components may be added in particular orders or that preparation may proceed separately from some components prior to combination into the final composition. For example, it may be beneficial to first dissolve certain antioxidants in solvents before their addition to the final composition.

*Effects of Antioxidant Compositions*

The antioxidant compositions of the invention may have any variety of positive effects. In one embodiment, they may be used to treat or prevent oral diseases. Oral diseases may include periodontal disease as well as other oral sores, lesions, ulcers or wounds. The antioxidant compositions of the invention may also generally be used to promote oral health, such as gingival health and to enhance wound healing at intra-oral surgical sites, including dental implant sites. Oral diseases treated or prevented by the antioxidant compositions of the invention may be the result of oxidative damage, such as resulting form tobacco, tooth whitener, or alcohol use, or they may be result of other types of damage.

Oral diseases treated or prevented using antioxidant compositions of the invention may also be the result of other diseases. For example, the decrease in salivary reduced-glutathione levels in patients with type 1 diabetes mellitus may have a role in periodontal destruction by predisposing tissues to oxidative stress. Diabetes-associated oxidative stress is a consequence of the production of free radicals and a reduced antioxidative capacity. (*See* Gumus P, et al. Salivary Antioxidants in Patients with Type 1 or 2 Diabetes Mellitus and Inflammatory Periodontal Disease: A case-Control Study. J. Periodontol. 80:1440-1446 (2009).) Accordingly, antioxidant compositions of the invention may be used to treat or prevent this diabetes-associated periodontal disease.

As used herein, the term "treat" refers not only to curing or substantially curing a disease, but also to decreasing the severity of one or more symptom or sign of the disease, causing one or more physiological effects that may lead to a decrease in the severity of the disease, halting or slowing the progression of the disease. As used herein "prevent" refers to avoiding the detectable occurrence of the disease or one or more of its symptoms.

According to one embodiment, antioxidant compositions may be used to treat or prevent diseases caused by various environmental factors, such as bleaching agents, tobacco use, smokeless tobacco, or alcohol. In particular, antioxidant compositions may be used to eliminate, reduce or reverse some negative oral effects of nicotine, such as reduced mobility of cells. The antioxidant compositions may also be used to eliminate, reduce or reverse some of the negative oral effects of ethanol or oral treatment products, such as hydrogen peroxide.

According to specific embodiments, the antioxidant compositions may treat or prevent various conditions by generally promoting healing. Promoting healing may benefit oral diseases caused by environmental oxidants as well as oral diseases caused by natural oxidative processes in healthy tissue or due to disease. Wound healing induced or aided by the antioxidant compositions of the invention may also treat or prevent oral diseases other than those resulting from oxidative damage, such as surgical wounds. Without limiting these embodiments to a specific mechanism of action, healing may be carried out primarily through mobile cells able to migrate to different locations to participate in healing. Such cells may include juvenile cells such as precursor cells or multipotent stem cells, as well as mobile adult cells, such as fibroblasts. Specifically, mobile cells may include human gingival fibroblasts or human periodontal ligament cells, as shown in the Examples below. Effects similar to those shown in the examples may be seen with other mobile cell types.

A periodontal disease may include infection or inflammation of the gingiva, periodontal ligament, teeth and or supporting bone. According to one embodiment, the antioxidant compositions of the invention may be used to treat or prevent periodontal disease such as periodontitis and gingivitis by treating a soft oral tissue. Patients with gingivitis may display red, swollen gums that bleed easily without substantial discomfort. Gingivitis may progress to periodontitis, for example where a bacterial plaque spreads under the gum line. Toxins produced by infecting bacteria may irritate the gingiva and may induce a chronic inflammatory response. Patients with periodontitis may present gingiva that have separated from the teeth, creating pockets that may be or may become infected. Progression of periodontitis may be marked by deepening pockets or destruction of gingiva or bone. Teeth may loosen and either fall out or require extraction.

In serious periodontal disease, the periodontal ligament, a soft tissue which helps anchor a tooth from its root to the underlying bone, may also become damaged. While this problem may be partially addressed by healing surrounding gingival tissue using gingival fibroblasts, it may also be treated or prevented by increasing the number or mobility of periodontal ligament cells, which may be able to directly repair damage to the periodontal ligament. Accordingly, antioxidant compositions of the invention may be able to decrease apoptosis in, increase proliferation of, or increase migration of periodontal ligament cells.

Oxidative damage can affect all cell types. Accordingly, the treatment or preventative effects of the antioxidant compositions are not limited to gingival fibroblasts or periodontal ligament cells in all embodiments of the invention. For example, similar effects regarding apoptosis, proliferation, or migration may be seen in other oral cells, particularly other oral cells involved in wound healing, such as fibroblasts and fibroblast precursors. As a consequence, the site of therapeutic or preventative benefit provided by antioxidant compositions may not be fixed to the cells of the epithelial layer that actually contact the antioxidant compositions. Therapeutic or preventative benefit may also be provided to underlying cells in addition to the fibroblasts and fibroblast precursors, for example the periodontal ligament, lamina propria and submucosal layers, including mesenchymal and periodontal ligament cells, and gingival fibroblasts.

In order to achieve therapeutic or preventative effects, antioxidants may be absorbed from the antioxidant compositions into the internal regions of oral soft tissues in some embodiments. However, in many cases of periodontal disease, it may be sufficient for the antioxidant compositions to merely contact the gingiva. In such instances, the ability of the antioxidant to reach and be absorbed by the gingival surfaces or by the tissues of the gingival sulcus, which is between the gingiva and tooth, may be sufficient.

Although the Examples present test data obtained with human cells, the antioxidant compositions are expected to have similar effects in other animals, such as other mammals, particularly domesticated mammals, that contain soft oral tissue subject to oral diseases, such as periodontal disease, because mechanisms of oxidative damage to such tissue and wound healing responses tend to be similar to those found in humans. Antioxidant compositions may be formulated by one of ordinary skill in the art, using the above disclosure, in appropriate veterinary forms. For example, antioxidant concentrations in compositions intended for some animals may be higher due to increased saliva production in such animals as compared to humans or to allow for less-frequent administration, minimizing the effort needed to use the product, or to account for an increased tendency by animals to swallow material placed in the mouth instead of allowing it to sit on the soft oral tissues. Antioxidant compositions may also be delivered in products, for example as coatings or components of chew toys or in feed or treats, that are unique to the veterinary market. Further, indications for administration of antioxidant compositions of the invention may be different for veterinary patients due to different environmental sources of oxidative damage. For example, although animals tend to not be frequently exposed to nicotine or alcohol, many animals may be exposed to other plant matter or food sources containing high levels of oxidants. Antioxidant compositions of the current invention may be administered to such animals after such types of exposure.

EXAMPLES

The present invention may be better understood through reference to the following examples. These examples are included to describe exemplary embodiments only and should not be interpreted to encompass the entire breadth of the invention.

*Example 1 - Effects of Antioxidant Compositions on Gingival Fibroblasts and Periodontal Ligament Cells and on Nicotine-Exposed Cells*

Three different combinations of three antioxidants were applied to gingival fibroblasts to determine the effects of these compositions on human gingival fibroblast (HGF) proliferation and migration and also to detect any toxic effects these compositions may have on gingival fibroblasts. Effects on human periodontal ligament cells (HPDL) were also studied.

Compositions were prepared as 40% w/v (total antioxidants) solutions in DMSO (e.g. a total of 400 mg antioxidants in 1 mL total volume) ($1.6 \times 10^{-3}$ M). Compositions were diluted with DMSO to achieve the lower concentrations of 4% w/v ($1.6 \times 10^{-4}$ M) and 0.4% w/v ($1.6 \times 10^{-5}$ M).

Phloretin was 98.6% pure, molecular weight 274 (Kaden Biochemcials, Hamburg, Germany). In compositions containing phloretin, it was present at a concentration of between $4.9 \times 10^{-3}$ M (for the 40% w/v composition) and $4.9 \times 10^{-5}$ M (for the 0.4% w/v composition).

Trans-ferulic acid was 99% pure, molecular weight 194 (Sigma-Aldrich, St. Louis, MO). In compositions containing trans-ferulic acid, it was present at a concentration of between $6.8 \times 10^{-3}$ M (for the 40% w/v composition) and $6.8 \times 10^{-5}$ M (for the 0.4% w/v composition). Tetrahydrocurcuminoids CG™ were 96.93% pure, molecular weight 372 (Sabinsa Corp., Piscataway, NJ). In compositions containing tetrahydrocurcuminoids, it was present at a concentration of between $3.6 \times 10^{-3}$ M (for the 40% w/v composition) and $3.6 \times 10^{-5}$ M (for the 0.4% w/v composition).

Resveratrol was 98% pure, molecular weight 228 (Lalilab, Inc. Durham, NC). In compositions containing resveratrol, it was present at a concentration of between 5.8 x 10$^{-3}$ M (for the 40% w/v composition) and 5.8 x 10$^{-5}$ M (for the 0.4% w/v composition).

In some tests conducted using three antioxidants, the total concentration was 0.5 M, in which case 0.166 M solutions of each antioxidant were combined to produce the final solution.

Certain tests were conducted using only two antioxidants, a 0.25 M solution of each antioxidant was prepared and combined to produce a final solution having a total of 0.5 M antioxidants.

Similarly, in tests using single antioxidants, the concentration was 0.5 M for each antioxidant.

*Trypan Blue Exclusion Test*

Basic effects of the antioxidant compositions on gingival fibroblast viability were determined using a trypan blue exclusion test. Human gingival fibroblast (HGF) cells were placed at 5 x 10$^4$ cells per well in a 24 well plate. The cells were exposed to the antioxidant compositions for 24 hours, 48 hours, 72 hours, or 96 hours. 4 % w/v, 4% w/v, and 0.4% w/v compositions were tested. The cells were then detached from the plate using 100 µL of 0.25% trypsin-EDTA for 5 minutes. The enzymatic reaction was stopped by adding 100 µL culture medium. Ten µL trypan blue was added to cells in 10 µL of solution and the cells were then counted under a light microscope with a hemacytometer.

Viable cells had a clear cytoplasm, whereas non-viable cells had a blue cytoplasm. Cell morphology during treatment was observed with phase-contrast microscopy. The percent of cells with a clear cytoplasm is recorded in Table 1.

*Table 1. Mean percentage of HGF cell viability*

| Antioxidant | 24 hr | 48 hr | 72 hr | 96 hr |
|---|---|---|---|---|
| 0.1% FBS | 95 | 100 | 100 | 100 |
| 0.1% DMSO | 100 | 100 | 100 | 94 |
| RFT 400 µg | 100 | 100 | 83 | 100 |
| PFR 400 µg | 100 | 100 | 100 | 94 |
| PFT 400 µg | 90 | 92 | 88 | 85 |
| 0.01% DMSO | 100 | 100 | 90 | 100 |
| RFT 40 µg | 100 | 100 | 100 | 100 |
| PFR 40 µg | 100 | 88 | 100 | 100 |
| PFT 40 µg | 100 | 100 | 100 | 95 |
| 0.001% DMSO | 100 | 100 | 88 | 92 |
| RFT 4 µg | 100 | 100 | 100 | 100 |
| PFR 4 µg | 100 | 100 | 88 | 92 |
| PFT 4 µg | 100 | 90 | 100 | 92 |

Results of similar tests for human periodontal ligament cells (HPDL) are presented in Table 2.

*Table 2. Mean percentage of HPDL cell viability*

| Antioxidant | 24 hr | 48 hr | 72 hr | 96 hr |
|---|---|---|---|---|
| 0.1% FBS | 100 | 98 | 99 | 99.5 |
| 0.1% DMSO | 100 | 100 | 98.5 | 100 |
| RFT 400 µg | 100 | 95.75 | 99.5 | 92 |
| PFR 400 µg | 98.5 | 95.5 | 96.5 | 97 |
| PFT 400 µg | 99.5 | 100 | 98.75 | 99 |
| 0.01% DMSO | 100 | 100 | 99.5 | 99.5 |
| RFT 40 µg | 100 | 90.5 | 97.75 | 92 |
| PFR 40 µg | 93.75 | 100 | 99.5 | 95 |
| PFT 40 µg | 99.75 | 100 | 96.66 | 96 |
| 0.001% DMSO | 100 | 98.5 | 95.5 | 95 |
| RFT 4 µg | 97.75 | 100 | 99.25 | 100 |
| PFR 4 µg | 100 | 100 | 99.5 | 100 |
| PFT 4 µg | 100 | 100 | 96.5 | 100 |

Neither test shows significant cell death as compared cells cultivated with control medium containing fetal bovine serum (FBS) or the carrier, DMSO.

*Cell Proliferation Assay*

Cell proliferation of gingival fibroblasts and periodontal ligament cells was measured using a Celltiter 96 Aqueous One Solution Cell Proliferation Assay (Promega, Madison, WI). Cells were incubated in 100 $\mu$L of cell culture medium at 10% to 80% confluence, then serum starved for 2 hours in 0.1% FBS medium. Antioxidant compositions or DMSO alone were added in either 10% FBS medium or 0.1% FBS medium. 40% w/v, 4% w/v, and 0.4% w/v antioxidant compositions were tested. Cells were incubated with antioxidant or controls for 24, hours, 48, hours, 72 hours, or 96 hours. Then, 20 $\mu$L of Aqueous One Solution Reagent was added for 1-4 hours at 37 °C in a humidified, 5% $CO_2$ atmosphere. Absorbance at 490 nm was measured using a plate reader. Results for human gingival fibroblasts (HGF) are presented in FIGURE 5 and Table 3. Growth for DMSO and antioxidant compositions are presented as % FBS control.

*Table 3. HGF Cell Proliferation*

| 24 hours antioxidant exposure | | | |
|---|---|---|---|
| **Antioxidant/Amount** | 400 $\mu$g | 40 $\mu$g | 4$\mu$g |
| 0.1% FBS | 100.0 | | |
| DMSO | 107.1 | 124.4 | 123.8 |
| RFT | 104.3 | 112.5 | 111.9 |
| PFR | 80.08 | 109.8 | 109.2 |
| PFT | 104.3 | 92.25 | 80.79 |
| 48 hours antioxidant exposure | | | |
| **Antioxidant/Amount** | 400 $\mu$g | 40 $\mu$g | 4$\mu$g |
| 0.1% FBS | 100.0 | | |
| DMSO | 84.9 | 92.98 | 89.11 |
| RFT | 64.3 | 92.54 | 95.47 |
| PFR | 55.12 | 87.72 | 88.35 |
| PFT | 79.31 | 84.84 | 79.74 |
| 72 hours antioxidant exposure | | | |
| **Antioxidant/Amount** | 400 $\mu$g | 40 $\mu$g | 4$\mu$g |
| 0.1% FBS | 100.0 | | |
| DMSO | 110.2 | 99.11 | 98.81 |
| RFT | 60.19 | 106.9 | 105.7 |
| PFR | 57.97 | 95.84 | 90.95 |
| PFT | 78.25 | 95 | 74.85 |
| 96 hours antioxidant exposure | | | |
| **Antioxidant/Amount** | 400 $\mu$g | 40 $\mu$g | 4$\mu$g |
| 0.1% FBS | 100.0 | | |
| DMSO | 112.8 | 113.8 | 101 |
| RFT | 64.79 | 116.1 | 125 |
| PFR | 67.67 | 107.7 | 121 |
| PFT | 89.93 | 109.9 | 86.11 |

Results for human periodontal ligament cells (HPDL) are presented in FIGURE 6 and Table 4. Growth for DMSO and antioxidant compositions are presented as % FBS control.

*Table 4. HPDLCell Proliferation*

| 24 hours antioxidant exposure | | | |
|---|---|---|---|
| **Antioxidant/Amount** | 400 μg | 40 μg | 4μg |
| 0.1% FBS | 100 | | |
| DMSO | 109.1 | 117 | 118.9 |
| RFT | 115.1 | 118.8 | 115.1 |
| PFR | 85.29 | 107.6 | 105 |
| PFT | 98.91 | 107 | 115.1 |
| 48 hours antioxidant exposure | | | |
| **Antioxidant/Amount** | 400 μg | 40 μg | 4μg |
| 0.1% FBS | 100 | | |
| DMSO | 121.4 | 111.6 | 109.1 |
| RFT | 101.4 | 120.6 | 105.2 |
| PFR | 92.8 | 101 | 98.33 |
| PFT | 95.69 | 103.2 | 97.86 |
| 72 hours antioxidant exposure | | | |
| **Antioxidant/Amount** | 400 μg | 40 μg | 4μg |
| 0.1% FBS | 100 | | |
| DMSO | 125.5 | 120.4 | 135.8 |
| RFT | 114 | 134.4 | 144.2 |
| PFR | 101.4 | 112.9 | 100.3 |
| PFT | 95.96 | 119.5 | 111.4 |
| 96 hours antioxidant exposure | | | |
| **Antioxidant/Amount** | 400 μg | 40 μg | 4μg |
| 0.1% FBS | 100 | | |
| DMSO | 123.2 | 104.1 | 126.4 |
| RFT | 92.8 | 116.3 | 129.3 |
| PFR | 82.78 | 111.2 | 101.7 |
| PFT | 86.74 | 124.6 | 111.1 |

For gingival fibroblasts, even low concentrations of the antioxidant compositions showed positive effects on proliferation at 24 hours. Positive proliferation effects for the 4% w/v and 0.4 % w/v continued to be seen even at 72 hours. Similar results were seen for periodontal ligament cells.

*Cell Morphology Assay*

A Giemsa staining assay was used to study the morphology and growth of human gingival fibroblast (HGF) cells in the presence of the antioxidant compositions. 24 well plates containing $20 \times 10^4$ HGF cells were washed with phosphate buffered saline (PBS) and fixed with methanol for 10 minutes at room temperature after 24 hours, 48 hours, 72 hours, or 96 hours of incubation with 40 % w/v, 4% w/v, or 0.4% w/v antioxidant compositions or control solution. The cells were than stained with 0.4% Giemsa solution by covering them for 15-20 minutes followed by rinsing with distilled water. Wells were allowed to dry then examined with a light microscope for overall cell morphology.

FIGURE 1 shows Giemsa-stained human gingival (HGF) cells after 48 hours of incubation, which are generally representative of the other time frames as well. The cells were mostly spindle-shaped with red cytoplasm. A round purple nucleus was observed at the center or margins of the cells. Overall, the antioxidant compositions did not appear to cause significant aberrations in the cell morphology. FIGURE 2 shows Giemsa-stained human periodontal ligament (HPDL) cells after 48 hours of incubation. The cells had a morphology similar to the HGF cells.

*Cell Migration Assay*

To test the effects of nicotine and antioxidant compositions on cell migration, human gingival fibroblast (HGF) or human periodontal ligament (HPDL) cells were grown to confluence in 4 quadrant dishes. They were then placed in 0.1% FBS medium and 6 mM nicotine for two hours. Some samples received 8 mM nicotine or 10 mM nicotine. Cells were then treated with a solution containing either the three antioxidants described above, or a 1:1 ratio of only two antioxidants, or only a single antioxidant. Cell migration was recorded using BioStation Live Cell Imaging (Nikon Instruments, Melvile, NY) every hour for ten hours, and data presented here is for 1 hour, 5 hours, or 10 hours.

FIGURE 7 shows the results of this assay for human gingival fibroblasts (HGF). As expected, nicotine had a deleterious effect on cell migration that increased with exposure to more concentrated nicotine. Treatment with antioxidants, however, restored cell migration to the levels seen in cells not treated with nicotine, or even increased cell migration beyond levels seen in the cells not treated with nicotine. These increased migration effects were even more pronounced at 5 and 10 hours after treatment. Similar results were seen for human periodontal ligament cells (HPDL) and are presented in FIGURE 8.

*Apoptosis Assay*

The effects of antioxidant compositions on apoptosis (a type of cell death) were studied in both human gingival fibroblasts (HGF) and human periodontal ligament (HPDL) cells using a Caspase 3 activity assay. Caspases are a family of proteases that mediate apoptosis. Caspase 3 activity was measured using a Caspase 3 colorimetric assay kit that is based on the hydrolysis of the peptide substrate Acetyl-Asp-Glu-Val-Asp p-nitroanilide (Ac-DEVD-pNA) by caspase 3, resulting in the release of the p-nitroaniline moiety (pNA). Control groups received DMSO only or a known inducer of apoptosis (Staurosporine, 1 $\mu$M) or the apoptosis inhibitor, DEVD-Z-VAD-FMK (50 $\mu$M).

To conduct the assay 50,000 cells/well were grown in a white view plate for 48 hours in the presence of antioxidant compositions $10^{-3}$ M (sample 1), $10^{-4}$ M (sample 2), or $10^{-5}$ M (sample 3). Cells were then washed with PBS and 50 $\mu$L of lysis buffer was added to each well. Cells were lysed using freeze-thaw cycles at -20 °C overnight. Cell lysates were then incubated for 15 minutes on ice and centrifuged at 15,000 g for 20 minutes. Then the amount of liberated pNA was detected, indicating the about of caspase activity.

Results for human gingival fibroblasts (HGF) are show in FIGURE 3 and Table 5.

*Table 5. Caspase Activity in Antioxidant-Treated HGF After 48 Hours of Treatment*

| Treatment/Test | 1 | 2 | 3 |
|---|---|---|---|
| 0.1% FBS | 18.76559133 | | |
| DMSO | 14.01651051 | 25.58172231 | 20.99218704 |
| RFT | 15.67472672 | 14.91173971 | 16.27819289 |
| PFR | 15.1056285 | 9.384800371 | 17.25924109 |
| PFT | 47.4452203 | 26.82855957 | 16.68865761 |
| st (1$\mu$m) | 208 | | |
| st +zvad | 35 | | |

Results for human periodontal ligament cells (HPDL) are shown in FIGURE 4 and Table 6.

*Table 6. Caspase Activity in Antioxidant-Treated HPDL After 48 Hours of Treatment*

| Treatment/Test | 1 | 2 | 3 |
|---|---|---|---|
| 0.1% FBS | 21.83672353 | | |
| DMSO | 24.56802275 | 13.21084864 | 17.78215223 |
| RFT | 19.73972003 | 18.17585302 | 17.27362205 |

(continued)

| Treatment/Test | 1 | 2 | 3 |
| --- | --- | --- | --- |
| PFR | 14.5641951 | 16.60104987 | 11.87937445 |
| PFT | 11.48567367 | 11.48567367 | 9.3667979 |
| st (1$\mu$m) | 152.962325 | | |
| st +zvad | 13.65 | | |

Overall the results indicate that the antioxidant compositions decreased the amount of apoptosis in treated cells.

*Example 2 - Effects of Antioxidants on Peroxide and Ethanol-Exposed Cells*

*General Techniques*

Human gingival tissues from healthy nonsmokers were collected with institutional review board approval and tissues were cultured in high glucose Dulbecco's modified eagles medium (DMEM), 10% FBS, and 1% antimycotic/antiobiotic. Tissues were incubated at 37°C in a humidified gas mixture (5% $CO_2$ and 95% air) and medium was changed every 24 hours. Human gingival fibroblasts (HGF) grew from the tissue after a week. Cells were passaged using 0.25% trypsin solutions and plated in new tissue culture flasks when confluent. Passages 3-12 were used in all of the experiments.
Human periodontal ligament fibroblasts (HPDL) isolated from freshly extracted human teeth were also used in relevant experiments. Culture conditions as described in HGF were similarly employed in the HPDL cells. In this experiment, 5 x 10$^3$ cells in 100 $\mu$L of culture medium were seeded into each well of a 96-well plate. After achieving 70% confluence, the cells were pre-treated with hydrogen peroxide ($H_2O_2$) or ethanol for 30 and 60 minutes. Bioactive antioxidant mixtures tested included: resveratrol, ferulic acid and tetrahydrocurcuminoids (RFT), phloretin, ferulic acid and resveratrol (PFR), phloretin, ferulic acid and tetrahydrocucurminoids (PFT). All mixtures were 10$^{-5}$ M (0.4% w/v) as described in Example 1.

*Pre-Treatments and Antioxidant Exposure*

A commercially available brand of hydrogen peroxide ($H_2O_2$) (3%) was obtained from Walgreens Co. (Deerfield Il.) Quantities of 0.001 % (290 $\mu$M), 0.00075% (223 $\mu$M) and 0.0005% (145 $\mu$M) $H_2O_2$ were used for the experiments. Cells were treated with hydrogen peroxide in serum-free media for 30 or 60 minutes. Cells were washed with PBS and treated with antioxidant mixtures (RFT, PFR, PFT) in 0.1% FBS medium (DMEM and 1% antimycotic/antiobiotic) for 24 hours.
Similar procedures were employed using ethyl alcohol pretreatments and antioxidant treatments. A pure ethyl alcohol was obtained from US Industrial Chemicals Company (Tuscola, IL). Different ethanol concentrations (15%, 10% and 5%) were used for dose response experiments for 30 or 60 minutes.
After pretreatment with ethanol or $H_2O_2$, the cells were rinsed and treated with 10$^{-5}$ M (0.4% wt/v) antioxidant mixture (RFT, PFR, PFT) for 24 hrs. HGF experiments had 3-6 replicate wells/treatment group and the experiments were repeated 3 times. HPDL experiments had 3-6 replicate wells/treatment group and the experiments were repeated 2 times.

*3-(4,5-dimethylthiazol-2-yl)-5-(3-carboxymethoxyphenyl)-2-(4-sulfophenyl)-2H-tetrazolium (MTS) Assay and Results*

Cell viability and survival were assessed by MTS colorimetric assay (3-[4,5-dimethylthiazol-2-yl]-5-[3-carboxymethoxyphenyl]-2-[4-sulfophenyl]-2H-tetrazolium) (Promega Corp., Madison, Wis). This quantitative assay detects living but not dead cells. After each pretreatment of $H_2O_2$ or ethyl alcohol and post antioxidant exposure, 20 $\mu$L of MTS reagent was added to each well and incubated for 2.5 hours at 37°C. The absorbance at 490 nm was measured using a SpectraMax microplate spectrophotometer (Molecular Devices, Sunnyvale, CA). The wells containing culture medium but no cells served as blanks.
The following calculations were used to determine the percentage for viability and survival:

a. Percent viable cells were expressed as; A/B, where A is absorbance at 490 nm with antioxidant compounds (RFT, PFR, PFT) and $H_2O_2$ or Ethanol pre-treatments; B absorbance at 490 nm with 0.1% FBS (control).
b. Percent Recovery/survival was based on the difference between percent viable cells of antioxidant-treated cells at 0.00075% $H_2O_2$ or 10% ethanol pre-treated cells.

Incubation of both HGF and HPDL cells in the presence of $H_2O_2$ resulted in a dose-dependent decrease of viable cells, with a noticeable effect at (0.00075%) $H_2O_2$ and (0.001%) $H_2O_2$ for 30 (FIGUREs 9 and 11, stippled bars) and 60 (FIGURES 10 and 12, stippled bars) minutes. $H_2O_2$ at 0.005% was not significantly different than control cell viability as measured by the MTS assay (FIGURES 9-12).

Ethanol also had a dose dependent effect on cell viability at 30 (FIGUREs 13 and 15, stippled bars) and 60 (FIGURES 14 and 16, stippled bars) minutes. All cells exposed to $H_2O_2$ or ethanol for 30 minutes and then treated with the antioxidant mixtures (RFT, PFR, PFT) had the same or increased viability (FIGURES 9, 11, 13, and 15). There was no difference in cell viability of cells treated with the lowest dose of $H_2O_2$ or ethanol (FIGURES 9-16). However, the highest doses of $H_2O_2$ killed the cells and they did not recover (FIGURES 9-12). Antioxidant compounds increased recovery or survival of cells exposed to $H_2O_2$ (0.00075%) and ethanol (10% or 15 %) (FIGUREs 9-11 and 13-16). One exception to the trend was HPDL cells treated with $H_2O_2$ for 1 hour, which did not recover with any of the antioxidant combination treatments (FIGURE 12), indicating that these cells were more sensitive to $H_2O_2$. There was a high percentage of recovery after antioxidant treatment; however, both cell types pretreated with 0.001% $H_2O_2$ were unable to recover after antioxidant treatment for 24 hours.

In particular, FIGURE 9 shows that all antioxidant compounds tested rescued HGF cells exposed to 0.00075% $H_2O_2$ for 30 min. HGF cells responded to increasing concentrations of $H_2O_2$ in a dose dependent manner (stippled bars). Antioxidants (RFT, PFR and PFT) increased cell survival at all concentrations except 0.001% $H_2O_2$, which killed the cells. A significant increase in the percentage of viable cells was observed in HGF cells pre-treated with 0.00075% $H_2O_2$.

FIGURE 10 shows that all antioxidant compounds tested rescued HGF cells exposed to 0.00075% $H_2O_2$ for 1 hour. HGF cells responded to increasing concentrations of $H_2O_2$ in a dose dependent manner (stippled bars). Antioxidants (RFT, PFR and PFT) increased cell survival at all concentrations except 0.001% $H_2O_2$, which killed the cells. A significant increase in the percentage of viable cells was observed in HGF cells pre-treated with 0.00075% H2O2. RFT was more effective than PFR or PFT at the lowest $H_2O_2$ concentration tested.

FIGURE 11 shows that all antioxidant compounds tested rescued HPDL cells exposed to 0.00075% or 0.001% $H_2O_2$ for 30 min. HPDL cells responded to increasing concentrations of $H_2O_2$ in a dose dependent manner (stippled bars). Antioxidants (RFT, PFR and PFT) increased cell survival at all concentrations. A significant increase in the percentage of viable cells was observed in HPDL cells pre-treated with 0.00075% or 0.001% $H_2O_2$.

FIGURE 12 shows that none of the antioxidant compounds tested rescued HPDL cells exposed to $H_2O_2$ for 1 hour. HPDL cells responded to increasing concentrations of $H_2O_2$ in a dose dependent manner (black bars). Antioxidants (RFT, PFR and PFT) either did not change or decreased cell survival at all concentrations except 0.001% $H_2O_2$, which killed the cells. Accordingly, as expected some high levels of oxidative damage may be too severe to be treated by antioxidants.

FIGURE 13 shows that all antioxidant compounds tested rescued HGF cells exposed to ethanol for 30 min. HGF cells responded to increasing concentrations of ethanol in a dose dependent manner (stippled bars). Antioxidants (RFT, PFR and PFT) increased cell survival at all concentrations. A significant increase in the percentage of viable cells was observed in HGF cells pre-treated with 5, 10 or 15% ethanol.

FIGURE 14 shows that all antioxidant compounds tested rescued HGF cells exposed to ethanol for 1 hour. HGF cells responded to increasing concentrations of ethanol in a dose dependent manner (stippled bars). Antioxidants (RFT, PFR and PFT) increased cell survival at 5 and 10% concentrations. The antioxidant groups pretreated with 10% ethanol showed a minimal increase in viable cells, again indicative that severly damaged cells may not be recoverable.

FIGURE 15 shows that all antioxidant compounds tested rescued HPDL cells exposed to ethanol for 30 min. HPDL cells responded to increasing concentrations of ethanol in a dose dependent manner (stippled bars). Antioxidants (RFT, PFR and PFT) increased cell survival at all concentrations. A significant increase in the percentage of viable cells was observed in HPDL cells pre-treated with 5, 10 or 15% ethanol. The cells pre-treated with 10 and 15% ethanol significantly increased the percentage of viable cells after antioxidant treatment. There was no difference between the antioxidant groups.

FIGURE 16 shows that all antioxidant compounds tested rescued HPDL cells exposed to ethanol for 1 hour. HPDL cells responded to increasing concentrations of ethanol in a dose dependent manner (stippled bars). Antioxidants (RFT, PFR and PFT) increased cell survival at all concentrations. The cells pre-treated with 10 and 15% ethanol had a smaller increase in the percentage of viable cells after antioxidant treatment than the 5% ethanol group. PFT appeared to be more effective at the highest concentration of ethanol.

*Example 3 - Mode of Action for Cell Movement*

*Cell Culture*

Both cell types tested (HGF and HPDL) were plated at 5 x 10$^4$ cells/well in a Lab-Tek II chamber glass slide with cover (Nalge Nunc International, Rochester, NY). Approximately 400 $\mu$l of cell medium suspension was added to each well. The cells were grown overnight until confluent. Cells were pretreated with nicotine (10 mM, 8 mM and 6 mM) for two hours and then treated with antioxidants (resveratrol, trans=ferulic acid, tetrahydrocurcuminoids CG™, or phloretin) in double or triple combinations at 0.4% w/v ($10^{-5}$M). Two hours later, a scratch wound assay was performed using a sterile 10 $\mu$l pipet tip. Cells were observed for 10 hours prior to immunohistochemical analysis for Rac-GTP.

*Immunofluorescence and Rac-GTP Quantification*

The cells were fixed with 4% paraformaldehyde for 30 minutes and then washed with sterile phosphate buffered saline (PBS) three times. The cells were blocked with 10% normal goat serum (NGS) for 1 hour at 4°C. After blocking procedures, the cells were washed with PBS three times. A specific primary antibody that recognizes Rac-GTP (1:500 dilution) was placed on the cells overnight at 4°C. Cells were washed with PBS three times. The secondary antibody (1:500 Alexa Fluor® 488 goat anti-mouse IgG (H+L) (Molecular Probes, Eugene, Oregon) was incubated for two hours at room temperature. The stained cells were washed with PBS three times. The Lab-Tek II wells were removed and the cells were cover-slipped with slowfade mounting medium. Analysis of the levels of Rac-GTP at the leading edge of the wound were determined using confocal microscopy. All treatments were recorded with the same settings on a Leica SP2 microscope. All images were taken from projected z stacks of the immunolabeled cells. Intensity of Rac-GTP immunofluorescence was determined using Nikon Elements software. All fluorescent pixels in a field (representing Rac-GTP detected by antibody) were included in a region of interest (ROI). The software then determined the mean fluorescence intensity within the region of interest.

Cell migration increased in response to antioxidants by increasing Rac-GTP expression. Nicotine-treated cells reduced migration and Rac-GTP in a dose dependent manner (FIGUREs 17 and 19, top row; FIGUREs 18 and 20, nic groups). Treatment with single, double and triple combinations of antioxidants increased the level of Rac-GTP activation in HGF (FIGURE 17) and HPDL (FIGURE 19) cells at the leading edge of the wound margin. The single antioxidant treatments appeared to restore the level of Rac-GTP to control (untreated 0.1% FBS) levels in both HGF (FIGURE 17, second row) and HPDL (FIGURE 19, second row) cells. However, the quantification of the pixel intensity demonstrated a 35% increase in intensity in the ferulic acid (Fa) treated group. The double and triple antioxidant treatments increased the labeling further, with an additive effect. The antioxidant-treated HPDL cells (FIGURE 19, PF and RFT) appeared to respond more than antioxidant treated HGF cells (FIGURE 17, PF, RFT). Pixel intensity analysis clearly demonstrated that the HPDL cells responded more vigorously to antioxidant treatment by increasing Rac-GTP expression up to 65%. (FIGURE 20).

FIGURES 17 and 18 specifically show that that Rac-GTP levels were more intense in antioxidant-treated HGF cells. Analysis of the levels of Rac-GTP at the leading edge of the wound in HGF was conducted using confocal microscopy. The images in FIGURE 17 are sample fields of the leading edge with the white areas showing the distribution of Rac-GTP staining. The black void area in the center is the in vitro wound in the monolayer of cells. FIGURE 18 expresses the results as a percentage of mean pixel intensity when compared with the untreated group (0.1% FBS). A dose dependent decrease of nicotine (6 mM, 8 mM, 10 mM) in Rac-GTP expression was observed. The counteractive effect of antioxidants (single, double, triple, $10^{-5}$M) was observed in the HGF cells treated with 6 mM nicotine. All three antioxidant groups (single, double, triple) showed an increased Rac-GTP expression as compared with the nicotine treated group (6 mM Nic). In the single antioxidant groups, trans-ferulic acid (Fa) increased the expression of Rac-GTP 35% compared to nicotine treated cells (6 mM Nic). In the double and triple antioxidant groups, PF and RFT increased Rac-GTP expression 45 and 40% over nicotine treated cells (6 mM Nic) respectively.

FIGURES 19 and 20 specifically show that Rac-GTP levels were more intense in antioxidant-treated HPDL cells. Analysis of the levels of Rac-GTP at the leading edge of the wound in HPDL was conducted using confocal microscopy. The images in FIGURE 19 are sample fields of the leading edge with the white areas showing the distribution of Rac-GTP staining. The black void area in the center is the in vitro wound in the monolayer of cells. FIGURE 20 expresses the results as a percentage of mean pixel intensity when compared with the untreated group (0.1% FBS). A dose dependent decrease of nicotine (6 mM, 8 mM, 10 mM) in Rac-GTP expression was observed. The counteractive effect of antioxidants (single, double, triple, $10^{-5}$M) was observed in the HPDL cells treated with 6 mM nicotine. All three antioxidant groups (single, double, triple) showed an increased Rac-GTP expression as compared with the nicotine treated group (6 mM Nic). In the single antioxidant groups, all antioxidants increased Rac-GTP expression compared to nicotine treated cells (6 mM Nic). In the double and triple antioxidant groups, PF and RFT increased Rac-GTP expression 66 and 60% over nicotine treated cells (6 mM Nic) respectively.

*Example 4 - Antioxidant Compositions*

Several specific antioxidant compositions have been prepared.

*Reference Formulation 1* - Preparation of gel containing resveratrol, trans-ferulic acid and tetrahydrocurcuminoids CG™

|  | Components | % w/w |
|---|---|---|
| **Part A** | Water | 76.29 |
|  | Xylitol | 10.00 |
|  | Sodium citrate | 0.154 |
|  | Citric acid monohydrate | 0.010 |
| **Part B** | Resveratrol | 0.114 |
|  | Trans-Ferulic acid | 0.097 |
|  | Tetrahydrocurcuminoids CG™ | 0.186 |
|  | Menthol | 0.100 |
|  | Thymol | 0.050 |
|  | Poloxamer 407 | 1.500 |
|  | PEG 600 | 2.000 |
|  | Propylene glycol | 2.000 |
|  | Ethanol | 5.000 |
| **Part C** | Ticalose ® CMC 2500 | 2.500 |

*Formulation 2* - Preparation of gel containing phloretin and trans-ferulic acid

|  | Components | % w/w |
|---|---|---|
| **Part A** | Water | 76.50 |
|  | Xylitol | 10.00 |
|  | Sodium Citrate | 0.154 |
|  | Citric acid monohydrate | 0.010 |
| **Part B** | Phloretin | 0.206 |
|  | Trans-ferulic acid | 0.146 |
|  | Menthol | 0.100 |
|  | Thymol | 0.050 |
|  | Poloxamer 407 | 1.500 |
|  | PEG 600 | 2.000 |
|  | Propylene glycol | 2.000 |
|  | Ethanol | 5.000 |
| **Part C** | Ticalose ® CMC 2500 | 2.500 |

The gels for Formulation 1 and Formulation 2 were prepared by dissolving xylitol, sodium citrate (if present), and citric acid in the water (Part A). The Part B components were dissolved at 60 °C and added to the prepared Part A. The mixture of Part A and Part B was stirred for 30 min and then ticalose (Part C) was added gradually. The mixture Part A, Part B, and Part C was stirred 2 hours and left overnight. The final gel was stirred at low rotation (50 rpm) for 1 hour.

*Formulation 3* - Preparation of film containing phloretin and trans-ferulic acid

|  | Components | % w/w |
|---|---|---|
| **Part A** | Water | 50.0 |
|  | Pullulan | 9.50 |
|  | Xanthan gum | 0.06 |
| **Part B** | Phloretin | 0.14 |
|  | Trans-ferulic acid | 0.10 |
|  | Menthol | 0.10 |
|  | Poloxamer 407 | 0.75 |
|  | PEG 600 | 0.65 |
|  | Propylene glycol | 1.00 |
| **Part C** | Water | 37.60 |
|  | Sodium citrate | 0.154 |
|  | Citric acid monohydrate | 0.010 |

The film for Formulation 3 was prepared by stirring the components of Part A to form a gel and storing the gel overnight. Part C was prepared by mixing the components. The components of Part B were stirred at 60°C for 30 min followed by addition of the prepared Part C. The mixture Part B and C was stirred for an additional 30 min. Next, the prepared mixture of Part B and Part C was added to the gel formed from Part A and mixed for 1 hour. The final mixture of Part A, Part B, and Part C was poured on smooth surface and dried at ambient temperature to form a film.

*Reference Formulation* 4 - Preparation of mouthwash containing resveratrol, trans-ferulic acid and tetrahydrocurcuminoids CG™

|  | Components | % w/w |
|---|---|---|
| **Part A** | Water | 81.74 |
|  | Ethanol | 10.00 |
|  | Sodium citrate | 0.154 |
|  | Citric acid monohydrate | 0.010 |
| **Part B** | Resveratrol | 0.057 |
|  | Trans-ferulic acid | 0.049 |
|  | Tetrahydrocurcuminoids CG™ | 0.093 |
|  | Menthol | 0.100 |
|  | Thymol | 0.050 |
|  | Poloxamer 407 | 0.750 |
|  | PEG 600 | 1.000 |
|  | Propylene glycol | 1.000 |
|  | Ethanol | 5.000 |

Formulation 4 was prepared by mixing the sodium citrate, citric acid, and ethanol in water (Part A). Components of Part B were dissolved at 60°C and added to Part A. The mixture of Part A and Part B was stirred for 1 hour.

*Formulation 5* - Preparation of oral spray containing phloretin and ferulic acid

|  | Components | % w/w |
|---|---|---|
| Part A | Water | 80.83 |
|  | Ethanol | 10.00 |
|  | Sodium citrate | 0.154 |
|  | Citric acid monohydrate | 0.010 |
| Part B | Phloretin | 0.206 |
|  | Trans-ferulic acid | 0.146 |
|  | Menthol | 0.100 |
|  | Thymol | 0.050 |
|  | Poloxamer 407 | 1.500 |
|  | PEG 600 | 1.000 |
|  | Propylene glycol | 1.000 |
|  | Ethanol | 5.000 |

Formulation 5 was prepared in generally the same manner as Formulation 4 then placed in spray containers.

*Formulation 6* - Preparation of film-forming gel containing phloretin and ferulic acid

|  | Components | % w/w |
|---|---|---|
| Part A | Phloretin | 0.206 |
|  | Trans-ferulic acid | 0.146 |
|  | Poloxamer 407 | 1.500 |
|  | PEG 600 | 1.000 |
|  | Ethanol | 2.000 |
| Part B | Film-forming gel | 94.15 |

Formulation 6 may be prepared by combining the Part A components then incorporating them into Part B. Application of the film-forming gel may provide a layer adhered to the oral mucosa that allows time-controlled release of phloretin and ferulic acid, which may increase the biological response to the antioxidants.

**Claims**

1.   An oral antioxidant composition comprising:

between 0.0001% and 5.0% w/w trans-ferulic acid;
between 0.0001 % and 5.0 % w/w phloretin; and
an orally pharmaceutically acceptable carrier,
wherein the pH of the oral antioxidant composition is at least 5.5
for use in the treatment or prevention of an oral disease by topical application to soft oral tissue.

**2.** The oral antioxidant composition for use of Claim 1, wherein the oral antioxidant composition has a pH of between 5.5 and 8.0.

**3.** The oral antioxidant composition for use of Claim 1, wherein the oral antioxidant composition has a total amount of antioxidant of less than 5% w/w.

**4.** The oral antioxidant composition for use of Claim 1, wherein the oral antioxidant composition is formulated as a paste, gel, rinse, spray, aerosol spray, syrup, powder, reconstitutable powder, tablet, gum, lipstick, lip balm, lozenge, dental tray, teeth whitening delivery vehicle, pharmaceutical delivery vehicle or dissolvable strip.

**5.** The oral antioxidant composition for use of Claim 1, further comprising at least one additional component selected from the group consisting of:
solvents, preservatives, moisturizers, bases, viscosity enhancers, surfactants, therapeutic additives, flavor enhancers, color enhancers, water, and any combinations thereof.

**6.** The oral antioxidant composition for use of Claim 1, wherein the oral disease is a periodontal disease.

**7.** The oral antioxidant composition for use of Claim 1, wherein the periodontal disease is gingivitis or periodontitis.

**8.** The oral antioxidant composition for use of Claim 1, wherein the soft oral tissue is oral mucosa, gingival, tongue tissue or lip tissue.

**9.** The oral antioxidant composition for use of Claim 1, wherein the soft oral tissue is the hard or soft palate.


**Patentansprüche**

**1.** Orale Antioxidans-Zusammensetzung umfassend:

0,0001 % bis 5,0 % w/w trans-Ferulasäure;
0,0001 % bis 5,0 % w/w Phloretin; und
einen oralen pharmazeutisch akzeptablen Träger,

wobei der pH-Wert der oralen Antioxidans-Zusammensetzung wenigstens 5,5 beträgt zur Verwendung bei der Behandlung oder Vorbeugung einer oralen Krankheit durch topische Verabreichung an orales Weichgewebe.

**2.** Orale Antioxidans-Zusammensetzung zur Verwendung gemäß Anspruch 1, wobei die Antioxidans-Zusammensetzung einen pH-Wert von 5,5 bis 8,0 aufweist.

**3.** Orale Antioxidans-Zusammensetzung zur Verwendung gemäß Anspruch 1, wobei die orale Antioxidans-Zusammensetzung eine Gesamtmenge von Antioxidans von weniger als 5 % w/w aufweist.

**4.** Orale Antioxidans-Zusammensetzung zur Verwendung gemäß Anspruch 1, wobei die orale Antioxidans-Zusammensetzung formuliert ist als Paste, Gel, Spülung, Spray, Aerosol-Spray, Sirup, Pulver, rekonstituierbares Pulver, Tablette, Gummi, Lippenstift, Lippenbalsam, Dragee, Zahnschiene, Einrichtung zur Verabreichung von Zahnbleichmittel; Einrichtung zur pharmazeutischen Verabreichung oder ablösbarer Streifen.

**5.** Orale Antioxidans-Zusammensetzung zur Verwendung gemäß Anspruch 1, weiterhin umfassend wenigstens eine zusätzliche Komponente ausgewählt aus der Gruppe bestehend aus: Lösungsmitteln, Konservierungsmitteln, Feuchtigkeitsspendern, Basen, Viskositätsverstärkern, Tensiden, therapeutischen Additiven, Geschmacksverstärkern, Farbverstärkern, Wasser und Kombinationen davon.

**6.** Orale Antioxidans-Zusammensetzung zur Verwendung gemäß Anspruch 1, wobei die orale Krankheit eine periodontale Krankheit ist.

**7.** Orale Antioxidans-Zusammensetzung zur Verwendung gemäß Anspruch 1, wobei die periodontale Krankheit Gingivitis oder Periodontitis ist.

**8.** Orale Antioxidans-Zusammensetzung zur Verwendung gemäß Anspruch 1, wobei das orale Weichgewebe Mundschleimhaut, Zahnfleisch, Zungengewebe oder Lippengewebe ist.

**9.** Orale Antioxidans-Zusammensetzung zur Verwendung gemäß Anspruch 1, wobei das orale Weichgewebe harter oder weicher Gaumen ist.

**Revendications**

**1.** Composition antioxydante buccale comprenant :

entre 0,0001 % et 5,0 % pds/pds d'acide trans-férulique ;
entre 0,0001 % et 5,0 % pds/pds de phlorétine ; et
un support pharmaceutiquement acceptable pour voie buccale,
sachant que le pH de la composition antioxydante buccale est d'au moins 5,5,
pour utilisation dans le traitement ou la prévention d'une maladie buccale par application topique à un tissu buccal mou.

**2.** La composition antioxydante buccale pour utilisation de la revendication 1, sachant que la composition antioxydante buccale a un pH compris entre 5,5 et 8,0.

**3.** La composition antioxydante buccale pour utilisation de la revendication 1, sachant que la composition antioxydante buccale a une quantité totale d'antioxydant de moins de 5 % pds/pds.

**4.** La composition antioxydante buccale pour utilisation de la revendication 1, sachant que la composition antioxydante buccale est formulée comme pâte, gel, lotion de rinçage, agent de pulvérisation, agent de pulvérisation en aérosol, sirop, poudre, poudre reconstituable, comprimé, gomme, fard à lèvres, pommade à lèvres, pastille, plateau dentaire, vecteur de blanchiment dentaire, vecteur pharmaceutique ou bande soluble.

**5.** La composition antioxydante buccale pour utilisation de la revendication 1, comprenant au moins un composant additionnel sélectionné dans le groupe constitué par : les solvants, les agents conservateurs, les humidificateurs, les bases, les rehausseurs de viscosité, les agents tensioactifs, les additifs thérapeutiques, les rehausseurs de goût, les rehausseurs de couleur, l'eau, et toute combinaison de ceux-ci.

**6.** La composition antioxydante buccale pour utilisation de la revendication 1, sachant que la maladie buccale est une maladie parodontale.

**7.** La composition antioxydante buccale pour utilisation de la revendication 1, sachant que la maladie parodontale est la gingivite ou la parodontite.

**8.** La composition antioxydante buccale pour utilisation de la revendication 1, sachant que le tissu buccal mou est une muqueuse buccale, un tissu gingival, un tissu de la langue ou un tissu des lèvres.

**9.** La composition antioxydante buccale pour utilisation de la revendication 1, sachant que le tissu buccal mou est le palais dur ou mou.

0.1% FBS      0.1% DMSO      0.01% DMSO      0.001% DMSO

0.4 mg RFT      0.04 mg RFT      0.004 mg RFT

0.4 mg PFR      0.04 mg PFR      0.004 mg PFR

0.4 mg PFT      0.04 mg PFT      0.004 mg PFT

FIG.1

FIG.2

FIG.3

FIG.4

FIG.5

FIG.6

FIG.7

FIG.8

FIG.9

FIG.10

FIG.11

FIG.12

FIG.13

FIG.14

FIG.15

FIG.16

FIG.17

Rac-GTP EXPRESSION IN
NICOTINE TREATED HGF CELLS

FIG.18

EP 2 370 069 B1

0.1% FBS          6 mM Nic          8 mM Nic          10 mM Nic

Fa                Phl                Res               Thc

PT                RF                PF

FT                RP                RT

RFT               PFR               PFT

# FIG.19

FIG.20

EP 2 370 069 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1504215 A
- JP 2004231602 A
- WO 0061162 A
- US 6572882 B
- US 6790869 B
- US 6923981 B

**Non-patent literature cited in the description**

- **GUMUS P et al.** Salivary Antioxidants in Patients with Type 1 or 2 Diabetes Mellitus and Inflammatory Periodontal Disease: A case-Control Study. *J. Periodontol.*, 2009, vol. 80, 1440-1446